(19) Europäisches Patentamt
European Patent Office
Office européen des brevets

(11) Veröffentlichungsnummer: **0 241 918 B1**

(12) **EUROPÄISCHE PATENTSCHRIFT**

(45) Veröffentlichungstag der Patentschrift: **27.05.92**

(51) Int. Cl.⁵: **C07D 213/89**, A61K 31/44, //C07D309/38

(21) Anmeldenummer: **87105537.2**

(22) Anmeldetag: **14.04.87**

Die Akte enthält technische Angaben, die nach dem Eingang der Anmeldung eingereicht wurden und die nicht in dieser Patentschrift enthalten sind.

# (54) 1-Hydroxy-2-pyridone, Verfahren zu ihrer Herstellung und Arzneimittel, die sie enthalten, sowie bei der Herstellung der 1-Hydroxy-2-pyridone gebildete Zwischenprodukte.

(30) Priorität: **18.04.86 DE 3613061**
**02.08.86 DE 3626211**

(43) Veröffentlichungstag der Anmeldung:
**21.10.87 Patentblatt 87/43**

(45) Bekanntmachung des Hinweises auf die Patenterteilung:
**27.05.92 Patentblatt 92/22**

(84) Benannte Vertragsstaaten:
**AT BE CH DE ES FR GB GR IT LI LU NL SE**

(56) Entgegenhaltungen:
**DE-A- 2 214 608**
**DE-A- 2 234 009**

(73) Patentinhaber: **HOECHST AKTIENGESELL-SCHAFT**
**Postfach 80 03 20**
**W-6230 Frankfurt am Main 80(DE)**

(72) Erfinder: **Lohaus, Gerhard, Dr.**
**Uhlandweg 4**
**W-6233 Kelkheim (Taunus)(DE)**
Erfinder: **Dittmar, Walter, Dr.**
**Uhlandstrasse 10**
**W-6238 Hofheim am Taunus(DE)**
Erfinder: **Hänel, Heinz, Dr.**
**Tannenwaldallee 80**
**W-6380 Bad Homburg(DE)**
Erfinder: **Raether, Wolfgang, Dr.**
**Falkensteinstrasse 6**
**W-6072 Dreieich(DE)**
Erfinder: **Reuschling, Dieter, Dr.**
**Beethovenstrasse 27**
**W-6308 Butzbach(DE)**
Erfinder: **Gröbel, Bengt-Thomas, Dr.**
**Im Hainpfad 10**
**W-6232 Bad Soden am Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 241 918 B1

## Beschreibung

Gegenstand der Erfindung sind neue 1-Hydroxy-2-pyridone der allgemeinen Formel I (siehe Patentanspruch 1), ihre Anwendung zur Bekämpfung hauptsächlich von Infektionen durch Pilze und Hefen sowie Arzneimittel, die diese Verbindungen enthalten; Erfindungsgegenstand sind weiterhin auch spezielle bei der Herstellung der neuen 1-Hydroxy-2-pyridone gebildete Zwischenprodukte.

Aus der DE-PS 22 34 009 sind Verbindungen der Formel II bekannt

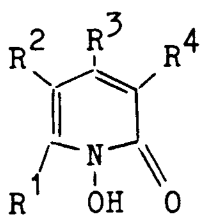

II

in der $R^1$ u.a. Aryloxyalkyl oder Arylmercaptoalkyl mit Alkyl von 1 - 4 C-Atomen bedeutet. Diese Reste werden spezifisch nur als Phenyloxymethyl oder Phenylmercaptomethyl erläutert. Außer Aryloxyalkyl oder Arylmercaptoalkyl kann $R^1$ nach der DE-PS 22 34 009 auch verschiedene andere Reste darstellen, wie Aryl, Aralkyl mit Alkyl von 1 - 4 C-Atomen, Arylalkenyl mit Alkenyl von 2 - 4 C-Atomen, Benzhydryl und Phenylsulfonylalkyl mit Alkyl von 1 - 4 C-Atomen. Soweit für diese Reste in der genannten Patentschrift Spezifizierungen angegeben werden, handelt es sich durchweg - mit Ausnahme des Arylrestes selbst, der auch als Naphthyl angegeben wird - nur um Phenylreste, die gegebenenfalls durch Alkylgruppen mit 1 - 4 C-Atomen, Alkoxygruppen mit 1 - 4 C-Atomen, Nitrogruppen, Cyangruppen oder Halogen substituiert sind.

Demgegenüber bezieht sich die Erfindung auf solche 1-Hydroxy-2-pyridonderivate, in denen der Substituent in 6-Position ($R^1$ in Formel II) ein aromatisches System beinhaltet, das mindestens 2 isolierte, gegebenenfalls substituierte aromatische Ringe enthält und über eine Oxymethylgruppe oder eine Thiomethylgruppe an den Pyridonrest gebunden ist, und die durch die allgemeine Formel I beschrieben werden.

Gegenstand der Erfindung sind also 1-Hydroxy-2-pyridone der allgemeinen Formel I (s. Patentanspruch 1), worin bedeuten

| | |
|---|---|
| $R^1$, $R^2$ und $R^3$, | die gleich oder verschieden sind, Wasserstoff oder niederes Alkyl mit 1 - 4 Kohlenstoffatomen, wobei $R^1$ und $R^3$ vorzugsweise Wasserstoff sind und $R^2$ vorzugsweise Methyl ist, |
| X | S oder vorzugsweise O, |
| Y | Wasserstoff oder bis zu 2 Halogenatome, nämlich Chlor und/oder Brom, |
| Z | eine Einfachbindung oder die zweiwertigen Reste O, S, $-CR_2-$ (R = H oder $C_1$-$C_4$-Alkyl) oder andere 2-wertige Reste mit 2 - 10 kettenförmig verknüpften C- und gegebenenfalls O- und/oder S-Atomen, wobei - wenn die Reste 2 oder mehr O- und/oder S-Atome enthalten - letztere durch mindestens 2 C-Atome voneinander getrennt sein müssen und wobei 2 benachbarte C-Atome auch durch eine Doppelbindung miteinander verknüpft sein können und die freien Valenzen der C-Atome durch H und/oder $C_1$-$C_4$-Alkylgruppen abgesättigt sind, |
| Ar | ein aromatisches Ringsystem mit bis zu zwei Ringen, das durch bis zu drei gleiche oder verschiedene Reste aus der Gruppe Fluor, Chlor, Brom, Methoxy, $C_1$-$C_4$-Alkyl, Trifluormethyl und Trifluormethoxy substituiert sein kann. |

In den Resten Z sind die C-Kettenglieder vorzugsweise $CH_2$-Gruppen. Wenn die $CH_2$-Gruppen durch $C_1$-$C_4$-Alkylgruppen substituiert sind, sind $CH_3$ und $C_2H_5$ bevorzugte Substituenten.

Beispielhafte Reste Z sind:

$-O-$, $-S-$, $-CH_2-$, $-(CH_2)_m-$ (m = 2-10), $-C(CH_3)_2-$, $-CH_2O-$, $OCH_2-$, $-CH_2S-$, $-SCH_2-$, $-SCH(C_2H_5)-$, $-CH=CH-CH_2O-$, $-O-CH_2-CH=CH-CH_2O-$, $-OCH_2CH_2O-$, $-OCH_2CH_2CH_2O-$, $-SCH_2CH_2CH_2S-$, $-SCH_2CH_2CH_2CH_2O-$, $-SCH_2CH_2OCH_2CH_2O-$, $-SCH_2CH_2OCH_2CH_2O-CH_2CH_2S-$, $-S-CH_2-C(CH_3)_2-CH_2-S-$, etc.

Der Begriff aromatisches Ringsystem umfaßt Phenyl und kondensierte Systeme wie Naphthyl, Tetrahydronaphthyl und Indenyl, sowie isolierte Systeme wie solche, die sich vom Biphenyl, Diphenylalkanen, Diphenyläthern und Diphenylthioäthern ableiten.

Wichtige Vertreter der durch die Formel I charakterisierten Verbindungsklasse sind z.B. 6-[4-(4-Chlorphenoxy)-phenoxymethyl]-1-hydroxy-4-methyl-2-pyridon, Schmp. 167 °C (1), 6-[4-(2,4-Dichlor-phenoxy)-phenoxymethyl]-1-hydroxy-4-methyl-2-pyridon, Schmp. 162 °C (2), 6-(Biphenylyl-4-oxymethyl)-1-hydroxy-4-methyl-2-pyridon, Schmp. 184 °C (3), 6-(4-Benzyl-phenoxymethyl)-1-hydroxy-4-methyl-2-pyridon, Schmp.

149 ° C (4), 6-[4-(2,4-Dichlorbenzyloxy)-phenoxymethyl]-1-hydroxy-4-methyl-2-pyridon, Schmp. 172 ° C (5), 6-[4-(4-Chlor-phenoxy)-phenoxymethyl]-1-hydroxy-3,4-dimethyl-2-pyridon, Schmp. 155 ° C (6), 6-[4-(2,4-Dichlorbenzyl)-phenoxymethyl]-1-hydroxy-3,4-dimethyl-2-pyridon, Schmp. 169 ° C (7) 6-[4-(Cinnamyloxy)-phenoxymethyl]-1-hydroxy-4-methyl-2-pyridon. Schmp. 179 ° C (8), 1-Hydroxy-4-methyl-6-[4-(4-trifluormethyl-phenoxy)-phenoxymethyl]-2-pyridon, Schmp. 149 ° C (9), 1-Hydroxy-4-methyl-6-[4-(naphth-1-yl-methoxy)-phenoxymethyl]-2-pyridon, Schmp. 179 ° C (10), 6-[4-(4-Chlor-phenoxy)-phenoxymethyl]-1-hydroxy-4,5-dimethyl-2-pyridon (11), 6-[4-(4-Chlorphenoxy)-phenoxymethyl)-phenoxymethyl]-1-hydroxy-4-methyl-2-pyridon, Schmp. 158 ° C (12), 6-[2,6-Dichlor-4-(naphth-2-yl-thiomethyl)-phenoxymethyl]-1-hydroxy-4-methyl-2-pyridon, Schmp. 138 ° C (13), 6-[2,6-Dichlor-4-(4-phenyl-phenoxymethyl)-phenoxymethyl]-1-hydroxy-4-methyl-2-pyridon, Schmp. 190 ° C (14), 6-[4-(4-Chlorbenzyloxy)-phenoxymethyl]-1-hydroxy-4-methyl-2-pyridon, Schmp. 173 ° C (15), 1-Hydroxy-4-methyl-6-[4-(4-trifluormethoxy-benzyloxy)-phenoxymethyl]-2-pyridon, Schmp. 143 ° C (16), 6-[4-(4-tert.Butyl-benzyloxy)-phenoxymethyl]-1-hydroxy-4-methyl-2-pyridon, Schmp. 181 ° C (17), 6-[2-(4-Chlor-benzyloxy)-phenoxymethyl]-1-hydroxy-4-methyl-2-pyridon, Schmp. 161 ° C (18), 1-Hydroxy-4-methyl-6-[2-(naphth-1-yl-methoxy)-phenoxymethyl]-2-pyridon, Schmp. 150 ° C (19), 1-Hydroxy-4-methyl-6-[3-(naphth-1-yl-methoxy)-phenoxymethyl]-2-pyridon, Schmp. 155 ° C (20), 6-[3-(4-Chlorbenzyloxy)-phenoxymethyl]-1-hydroxy-4-methyl-2-pyridon, Schmp. 149 ° C (21), 6-[4-(4-Chlor-phenoxy)-phenoxymethyl]-1-hydroxy-2-pyridon, Schmp. 180 ° C (22), 6-[2,6-Dichlor-4-(4-chlor-phenoxy)-phenoxymethyl]-1-hydroxy-4-methyl-2-pyridon, Schmp. 150 ° C (23), 6-(4-Benzyloxy-2,6-dichlorphenoxymethyl)-1-hydroxy-4-methyl-2-pyridon, Schmp. 161 ° C (24), 6-(2,6-Dichlor-4-phenyl-phenoxymethyl)-1-hydroxy-4-methyl-2-pyridon, Schmp. 195 ° C (25), 6-[4-(4-Brom-2-chlor-phenoxy)-phenoxymethyl]-1-hydroxy-4-methyl-2-pyridon, Schmp. 174 ° C (26), 1-Hydroxy-4-methyl-6-[4-(3,4,5-trimethoxy-benzyloxy)-phenoxymethyl]-2-pyridon, Schmp. 154 ° C (27), 6-[4-(2,4-Dichlorbenzyl)-phenoxymethyl]-1-hydroxy-4-methyl-2-pyridon, Schmp. 173 ° C (28), 6-[2,6-Dibrom-4-(4-chlor-phenoxy)-phenoxymethyl]-1-hydroxy-4-methyl-2-pyridon (29), 6-(2,6-Dibrom-4-phenyl-phenoxymethyl)-1-hydroxy-4-methyl-2-pyridon (30), 6-(2-Brom-4-phenyl-phenoxymethyl)-1-hydroxy-4-methyl-2-pyridon, Schmp. 245 ° C (31), 6-(2-Brom-6-chlor-4-phenyl-phenoxymethyl)-1-hydroxy-4-methyl-2-pyridon (32), 6-[4-(4-Fluorphenoxy)-phenoxymethyl]-1-hydroxy-4-methyl-2-pyridon, Schmp. 151 ° C (33), 6-[3-(4-Chlor-phenylthio)-phenoxymethyl]-1-hydroxy-4-methyl-2-pyridon (34), 1-Hydroxy-4-methyl-6-[3-(naphth-1-yl-methylthio)-phenoxymethyl]-2-pyridon, Schmp. 144 ° C (35), 1-Hydroxy-4-methyl-6-[3-(naphth-1-yl-methoxy]-phenylthio-methyl]-2-pyridon, Schmp. 163 ° C (36), 1-Hydroxy-4-methyl-6-(2-phenylphenoxymethyl)-2-pyridon, Schmp. 179 ° C (37), 6-(2-Benzylphenoxymethyl)-1-hydroxy-4-methyl-2-pyridon, Schmp. 155 ° C (38), 1-Hydroxy-3,4-dimethyl-6-[3-(naphth-1-yl-methylthio)-phenoxymethyl]-2-pyridon, Schmp. 143 ° C (39), 6-(2,4-Dibrom-6-phenyl-phenoxymethyl)-1-hydroxy-4-methyl-2-pyridon, Schmp. 130 ° C (40), 6-[4-(4-(4-Chlor-phenoxy)-phenoxy)-phenoxymethyl]-1-hydroxy-4-methyl-2-pyridon, Schmp. 100 ° C (41), 6-[3-(4-Chlor-benzyloxy)-phenylthiomethyl]-1-hydroxy-4-methyl-2-pyridon, Schmp. 94 ° C (42), 6-[4-(4-Chlor-phenylthio)-phenoxymethyl]-1-hydroxy-4-methyl-2-pyridon, Schmp. 158 ° C (43), 1-Hydroxy-6-[4-(4-methoxy-phenylthio)-phenoxymethyl]-4-methyl-2-pyridon, Schmp. 162 ° C (44), 1-Hydroxy-4-methyl-6-[3-(phenoxyethoxy)-phenoxymethyl]-2-pyridon, Schmp. 148 ° C (45), 6-[4-(4-Chlorphenoxy-propoxy)-phenoxymethyl]-1-hydroxy-4-methyl-2-pyridon, Schmp. 162 ° C (46), 6-[3-(4-Chlorphenylthio-propylthio)-phenoxymethyl]-1-hydroxy-4-methyl-2-pyridon, Schmp. 102 ° C (47), 6-[3-(4-Chlorphenylthio-butoxy)-phenoxymethyl]-1-hydroxy-4-methyl-2-pyridon, Schmp. 104 ° C (48), 6-[3-(4-Chlorphenylthioethoxyethoxy)-phenoxymethyl]-1-hydroxy-4-methyl-2-pyridon, Schmp. 98 ° C (49), 6-[4-($\alpha$,$\alpha$-Dimethyl-4-methoxy-benzyl)-phenoxymethyl]-1-hydroxy-4-methyl-2-pyridon, Schmp. 156 ° C (50), 6-⟨3-[1-(4-Chlorphenylthio)-2,2-dimethyl-prop-3-yl-thio]-phenoxymethyl⟩-1-hydroxy-4-methyl-2-pyridon, Schmp. 134 ° C (51), 6-⟨4-[1-(4-Chlorphenyl)-but-2-en-4-yl-oxy]-phenoxymethyl⟩-1-hydroxy-4-methyl-2-pyridon, Schmp. 167 ° C (52), 6-[3-(4-Chlorphenylthio-ethoxyethoxyethylthio)-phenoxymethyl]-1-hydroxy-4-methyl-2-pyridon, Schmp. 95 ° C (53), 6-⟨4-[1-⟨4-Chlorphenyl)-pent-5-yl]-phenoxymethyl⟩-1-hydroxy-4-methyl-2-pyridon, Schmp. 159 ° C (54).

Die Herstellung der erfindungsgemäßen Verbindungen kann auf verschiedenen, an sich bekannten Wegen erfolgen, z.B. durch Umsetzung von 6-Halogenmethyl-2-pyronen der Formel III (siehe Patentanspruch 9) mit gegebenenfalls geeignet substituierten Phenolen oder Thiophenolen der Formel IV (siehe Patentanspruch 9) und Umwandlung der gebildeten Aryloxymethyl-pyrone bzw. Arylthiomethyl-pyrone der Formel V (s. Patentanspruch 9) in die Hydroxypyridone durch Einwirkung von Hydroxylamin. Die Alkylierungen werden zweckmäßig in protischen oder aprotischen Lösungsmitteln, wie Methanol, Äthanol, Isopropanol, Aceton, Acetonitril, Äthylenglykol-dimethyläther, Diäthylenglykoldimethyläther, Dimethylformamid oder Dimethylsulfoxid durchgeführt, wobei die aprotischen Lösungsmittel bevorzugt sind. Zur Bindung des freiwerdenden Halogenwasserstoffs werden anorganische oder organische Basen wie Natrium- oder Kaliumhydroxid, Natrium-, Kalium- oder Calciumcarbonat, Triäthylamin, Tributylamin, Pyridin, 4-Dimethylaminopyridin, Diaza-bicyclononan, N-Methylpiperidin u.a. in mindestens äquivalenten Mengen eingesetzt. Die

Reaktionstemperaturen liegen im allgemeinen zwischen Raumtemperatur und ca. 80 °C; in besonderen Fällen können jedoch auch deutlich höhere oder tiefere Temperaturen wie etwa 110 °C oder 0 °C vorteilhaft sein.

Zur Umwandlung der 2-Pyrone in die 1-Hydroxy-2-pyridone setzt man das Hydroxylamin im allgemeinen in Form seiner Salze mit anorgansichen oder organischen Säuren, vorzugsweise der Salz-, Schwefel- oder Essigsäure, in Gegenwart von mindestens etwa einem Äquivalent einer Base, bezogen auf das Hydroxylammonium-Salz, um. Die Menge des Hydroxylaminsalzes beträgt mindestens etwa 1 Mol, bezogen auf das eingesetzte Pyron; jedoch ist es zur Erhöhung der Reaktionsgeschwindigkeit und der Ausbeute günstig, einen Überschuß einzusetzen, etwa zwischen 2 und 10 Mol, bezogen auf ein Mol, und zudem diese Menge in mehreren Portionen während der Umsetzungsdauer zuzugeben. Als Basen für diese Reaktion kommen sowohl organische als auch anorganische Basen in Betracht. Bevorzugte organische Basen sind Aminopyridin(-Derivate) und Imidazol(-Derivate) wie 2-Aminopyridin, 2-Amino-picolin, 2-Methylamino-pyridin, Imidazol und 2-Methyl-imidazol; bevorzugte anorganische Basen sind die Carbonate und/oder Bicarbonate der Alkalimetalle ($Li_2CO_3$, $Na_2CO_3$, $K_2CO_3$, $NaHCO_3$, $KHCO_3$, $Rb_2CO_3$, $CsHCO_3$, etc.). Von den genannten anorganischen Basen sind die Carbonate und Bicarbonate des Natriums und Kaliums,vor allem das $Na_2CO_3$, besonders geeignet.

Die organischen Basen werden im allgemeinen in Mengen zwischen ca. 1 und 20 Mol, vorzugsweise zwischen ca. 3 und 10 Mol je Mol des eingesetzten Pyrons verwendet und können gleichzeitig als Lösungsmittel dienen; damit ist normalerweise auch die Bedingung erfüllt, daß mit Bezu auf das eingesetzte Hydroxylammoniumsalz mindestens etwa 1 Basenäquivalent zugegen ist.

Natürlich können auch Mischungen dieser Basen eingesetzt werden, etwa um den Schmelzbereich des Systems herabzusetzen, wenn bei tiefen Temperaturen gearbeitet werden soll. Im allgemeinen liegen die Reaktionstemperaturen hier zwischen ca. 20 °C und 150 °C, vorzugsweise zwischen ca. 50 °C und 100 °C.

Im Falle des Einsatzes der anorganischen Basen wird - wie bei den organischen Basen - zweckmäßig eine der verwendeten Menge an Hydroxylammoniumsalz mindestens etwa äquivalente Menge zugesetzt. Pro Mol Hydroxylammoniumchlorid ist beispielsweise mindestens 1/2 Mol $Na_2CO_3$ oder 1 Mol $NaHCO_3$ zu verwenden. Auch die anorganischen Basen können sowohl einzeln als auch in beliebiger Mischung zum Einsatz gelangen.

Zur Durchführung der Variante mit den anorganischen Basen mischt man vorteilhaft das 2-Pyron mit dem Hydroxylammoniumsalz - hier bevorzugt mit dem Hydroxylammoniumsulfat - und dem Alkalicarbonat und/oder -hydrogencarbonat und erwärmt den erhaltenen Kristallbrei, bis das Pyron weitestgehend umgesetzt ist; das entstandene 2-Pyridon kann nach Abtrennung der anorganischen Salze direkt - oder besser noch als Salz einer organischen Base, z.B. als Ethanolaminsalz - isoliert werden.

Die Temperatur, bei der diese Variante ausgeführt wird, sollte ca. 120 °C keinesfalls überschreiten. Sie liegt zweckmäßig über ca. 50 °C und vorzugsweise zwischen etwa 60 und 105 °C.

Es ist auch möglich, sowohl bei der Variante mit organischen als auch derjenigen mit anorganischen Basen, inerte Lösungs-bzw. Verdünnungsmittel zuzusetzen. Im allgemeinen ist dies zwar nicht erforderlich , kann jedoch im Einzelfall Vorteile bieten. Wenn Lösungs- bzw. Verdünnungsmittel zugesetzt werden, geschieht dies im allgemeinen nur in kleinen Mengen, meistens bis zu ca. 50 Gew.-% des gesamten Reaktionsansatzes. Bevorzugt beträgt die Menge ca. 3 bis 15 Gew.-%.

Die Lösungs- oder Verdünnungsmittel können polar oder unpolar, mit Wasser mischbar oder nicht mischbar sein. Z.B. können folgende Substanzen verwendet werden: Wasser, niedermolekulare Alkohole wie Methanol, Ethanol, Isopropanol, Ethylenglykol, Ethylenglykol-monomethylether und Propylenglykol, Säureamide wie Dimethylformamid und Diethylformamid, Ether wie Diisopropylether, chlorierte Kohlenwasserstoffe wie Chlorbenzol, Nitrile wie Acetonitril, Kohlenwasserstoffe aliphatischer, cycloaliphatischer oder aromatischer Natur.

Die Herstellung der 6-Halogenmethyl-2-pyrone, insbesondere der Chlorverbindungen, kann z.B. in der in Chemische Berichte 100 (1967), S. 658 beschriebenen Weise erfolgen.

Eine andere Synthesemöglichkeit für die Hydroxypyridone besteht in der Seitenkettenhalogenierung von 2-Halogen-6-picolinen zu 2-Halogen-6-halogenmethylpyridinen der Formel VI (siehe Patentanspruch 9), Umsetzung der Halogenmethylgruppe mit gegebenenfalls geeignet substituierten Phenolen, Oxidation zum N-Oxid und Verseifung des kernständigen Halogens auf direktem oder indirektem Wege. Die Umsetzung der Halogenmethylgruppe mit den Phenolen wird vorzugsweise unter Bedingungen durchgeführt, wie sie oben für die Reaktion der Halogenmethyl-pyrone mit Phenolen beschrieben sind. Für die Umwandlung der Pyridine in ihre N-Oxide verwendet man anorganische oder organische Oxidationsmittel wie Wasserstoffperoxid, Perameisensäure, Peressigsäure, Perbenzoesäure, 3-Chlor-perbenzoesäure, tert.-Butylhydroperoxid, und arbeitet gegebenenfalls unter Katalyse durch eine starke Säure wie Schwefelsäure, Perchlorsäure, Toluolsulfonsäure, Trifluoressigsäure und Trifluormethansulfonsäure, vorzugsweise zwischen Raumtemperatur und ca. 100 ° C. Die Verseifung des kernständigen Halogens kann direkt erfolgen, z.B. durch Umsetzung mit Basen wie Natriumhydroxid, Kaliumhydroxid oder Bariumhydroxid oder indirekt über eine Verätherung mit einem seinerseits wieder leicht abspaltbaren Alkohol wie tert.-Butanol oder 2-Methoxyäthanol.

Typische Verfahrensweisen für die Herstellung der erfindungsgemäßen Verbindungen der allgemeinen Formel I werden durch die nachfolgenden Beispiele erläutert.

Soweit nach einem der vorstehenden Verfahren Zwischenprodukte erhalten werden, die in Ar oder dem Pyronring noch reaktionsfähige Substituenten enthalten, lassen sich über diese noch weitere Gruppen einführen, soweit diese den Definitionen $R^1$ bis $R^3$ und den für Ar angegebenen Substituenten entsprechen. Z.B. kann eine freie Hydroxy- oder Mercaptogruppe nachträglich veräthert werden, oder eine Hydroxymethylgruppe, z.B. gebildet durch Reduktion einer Aldehydgruppe, kann in eine Halogenmethylgruppe umgewandelt und das Halogen dann wieder nucleophil mit einem Phenol oder Thiophenol ausgetauscht werden. Analog lassen sich auch Pyridinderivate, die bei der Umsetzung der Dihalogenpicoline V mit den Phenolen anfallen, bzw. die daraus durch Oxidation erhaltenen N-Oxide, die noch reaktionsfähige Substituenten enthalten, zu anders gearteten Substitutionsprodukten umsetzen.

Gegenstand der Erfindung sind auch die im Anspruch 17 genannten Verbindungen der Formel V, die sich als Zwischenprodukte eignen.

Die erfindungsgemäßen Verbindungen der Formel I haben hervorragende topische antimykotische Eigenschaften mit einem breiten Wirkungsspektrum gegenüber pathogenen Pilzen, wie Dermatophyten (Fadenpilze) und solche Pilze, die sowohl die Haut als auch die Schleimhaut befallen, wie Hefen (z.B. Candida spp.), sowie Schimmelpilze (z.B. Aspergillus niger). Sie können deshalb zur Bekämpfung von Infektionen durch diese Erreger in der Human- und Veterinärmedizin, z.B. an Haustieren, wie Hunden, Katzen, Vögeln und Nutztieren, wie Wiederkäuern, Pferden und Schweinen eingesetzt werden. Die Anwendung kann als freie Hydroxypyridone oder in Form ihrer physiologisch verträglichen Salze mit anorganischen oder organischen Basen (z.B. mit NaOH, KOH, $Ca(OH)_2$, $NH_3$, $H_2NCH_2CH_2OH$, etc.) in den für die Bekämpfung von Pilzen üblichen Zubereitungsformen wie Lösungen, Suspensionen, Cremes, Salben, Puder oder Suppositorien (Ovula) erfolgen. Die neuen Präparate zeichnen sich insbesondere durch ihre hohe Fungizidie sowie eine lange Verweildauer am Infektionsort aus und sind darin Standardpräparaten des Handels überlegen, wie in den nachfolgend beschriebenen vergleichenden Untersuchungen gezeigt wird. Zusätzlich haben diese Verbindungen antibakterielle und antivirale Wirkungen, z.B. gegen Herpes-Viren.

## Beispiele

### 1: 6-[4-(2,4-Dichlor-benzyl)-phenoxymethyl]-1-hydroxy-3,4-dimethyl-2-pyridon (Verbindung 7)

Man löste 19,85 g 6-Chlormethyl-3,4-dimethyl-2-pyron (Verbindung A) und 25,3 g 4-(2,4-Dichlor-benzyl)-phenol in 70 ml Dimethylformamid, fügte 20 g fein gemahlenes Kaliumcarbonat hinzu und rührte 48 Stunden bei Raumtemperatur. Dann gab man 200 ml Methylenchlorid und 500 ml Wasser hinzu, trennte die Schichten, wusch die organische Phase zweimal mit je 100 ml Wasser, trocknete und dampfte im Vakuum der Wasserstrahlpumpe ein. Den dünnschichtchromatographisch fast einheitlichen Rückstand von 42,7 g erwärmte man mit 200 g 2-Amino-pyridin 56 Stunden auf 75 ° C und gab während der ersten 41 Stunden in 5 Portionen insgesamt 41,7 g Hydroxylamin-hydrochlorid zu. Man fügte dann 250 ml Methylenchlorid zu, wusch einmal mit verdünnter Salzsäure und zweimal mit Wasser, trocknete die organische Phase und destillierte das Lösungsmittel unter vermindertem Druck ab. Den Rückstand von 39,7 g kristallisierte man aus Äthylenglykolmonomethyläther um und erhielt 32,5 g reines Hydroxypyridon vom Schmelzpunkt 169 ° C.

2 bis 19: Auf die gleiche Weise wie im Beispiel 1 erhielt man, ausgehend von 4-(4-Chlor-phenoxy)-phenol und A die Verbindung 6, aus 3-(Naphth-1-yl-methylthio)-phenol und A die Verbindung 39, aus 4-(4-Chlor-phenoxy)-phenol und 6-Chlormethyl-4-methyl-2-pyron (Verbindung B) die Verbindung 1, aus 4-(2,4-Dichlor-phenoxy)-phenol und B die Verbindung 2, aus 4-(4-Trifluormethyl-phenoxy)-phenol und B die Verbindung 9, aus 2,6-Dichlor-4-(4-chlor-phenoxy)-phenol und B die Verbindung 23, aus 2,6-Dichlor-4-phenyl-phenol und B die Verbindung 25, aus 4-(4-Fluor-phenoxy)-phenol und B die Verbindung 33, aus 3-(4-Chlor-phenylthio)-phenol und B die Verbindung 34, aus 3-(Naphth-1-yl-methylthio)-phenol und B die Verbindung 35, aus 2,4-Dibrom-6-phenyl-phenol und B die Verbindung 40, aus 4-[4-(4-Chlor-phenoxy)-phenoxy]-phenol und B die Verbindung 41, aus 4-(4-Chlor-phenylthio)-phenol und B die Verbindung 43, aus 4-Benzyl-phenol und B die Verbindung 4, aus 2-Benzyl-phenol und B die Verbindung 38, aus 4-Phenyl-phenol und B die Verbindung 3, aus 4-(4-Brom-2-chlor-phenoxy)-phenol und B die Verbindung 26 sowie aus 4-[1-(4-Chlorphenyl)-pent-5-yl]-phenol und B die Verbindung 54.

20: 1-Hydroxy-4-methyl-6-[4-(naphth-1-yl-methoxy)-phenoxymethyl]-2-pyridon (Verbindung 10)

Man rührte eine Mischung von 100 g 6-Chlormethyl-4-methyl-2-pyron, 210 g Hydrochinon, 132 g Kaliumcarbonat und 400 ml Dimethylformamid 72 Stunden bei Raumtemperatur, versetzte mit Wasser, neutralisierte mit Salzsäure, saugte die Ausfällung ab, wusch mit Wasser und trocknete. Durch Behandeln mit Methylenchlorid und anschließendes Umkristallisieren aus Acetonitril erhielt man 68 g praktisch reines 6-(4-Hydroxy-phenoxymethyl)-4-methyl-2-pyron vom Schmp. 179 °C. 4,8 g dieser Verbindung rührte man mit 4 g 1-Chlormethylnaphthalin, 20 ml Dimethylformamid und 8 g Kaliumcarbonat 72 Stunden bei Raumtemperatur, versetzte dann mit verdünnter Natronlauge, nahm mit Methylenchlorid auf, wusch die organische Phase mit Wasser, trocknete und destillierte das Lösungsmittel ab. Den Rückstand von 7,0 g chromatographierte man in Methylenchlorid über eine Säule mit Kieselgel und erhielt 4,6 g reines 4-Methyl-6-[4-(naphth-1-yl-methoxy)-phenoxymethyl]-2-pyron vom Schmp. 132 °C. Dieses Produkt erwärmte man mit 15 g 2-Amino-pyridin auf 75 °C und gab innerhalb von 32 Stunden unter Rühren 7 g Hydroxylaminhydrochlorid in 4 Portionen zu. Nach insgesamt 42 Stunden Reaktionsdauer nahm man mit Methylenchlorid auf, wusch mit verdünnter Salzsäure und Wasser, trocknete, destillierte das Lösungsmittel ab und kristallisierte den Rückstand aus Acetonitril um. Man isolierte 1,9 g der reinen Verbindung 10 vom Schmp. 179 °C.

21-30: Auf die gleiche Weise wie im Beispiel 20 erhielt man durch die Alkylierung des Zwischenproduktes 6-(4-hydroxy-phenoxymethyl)-4-methyl-2-pyron mit den Chloriden 2,4-Dichlor-benzylchlorid, Cinnamylchlorid, 4-Chlor-benzylchlorid, 4-Trifluormethoxy-benzylchlorid, 4-tert.-Butylbenzylchlorid, 3,4,5-Trimethoxy-benzylchlorid, 1-Chlor-3-(4-chlorphenoxy)-propan und 1-Chlor-4-(4-chlorphenoxy)-buten-(2) und Umwandlung der so erhaltenen Pyrone in die Hydroxypyridone die Verbindungen 5, 8, 15, 16, 17, 27, 46 und 52. Verwendung von Brenzkatechin anstelle von Hydrochinon und Alkylierung mit Naphth-1-yl-methylchlorid führte zur Verbindung 19, Resorcin und 4-Chlor-benzylchlorid zur Verbindung 21.

31: 6-[2,6-Dichlor-4-(naphth-2-yl-thiomethyl)-phenoxymethyl]-1-hydroxy-4-methyl-2-pyridon (Verbindung 13)

Man löste 4,8 g Natrium und 42 g 3,5-Dichlor-4-hydroxybenzaldehyd in 250 ml Methanol, destillierte das Lösungsmittel unter vermindertem Druck ab, nahm mit 200 ml Dimethylformamid auf, fügt 32 g 6-Chlormethyl-4-methyl-2-pyron hinzu und ließ 3 Tage bei Raumtemperatur reagieren. Anschließend destillierte man das Dimethylformamid unter vermindertem Druck weitgehend ab, setzte Methanol hinzu und isolierte durch Abkühlen und Einengen der Mutterlauge in mehreren Fraktionen insgesamt 44 g 6-(2,6-Dichlor-4-formyl-phenoxymethyl)-4-methyl-2-pyron vom Schmp. 180 °C. 34,5 g dieser Verbindung reduzierte man in einer Mischung aus 250 ml Tetrahydrofuran und 100 ml Methanol mit 1,5 g Natriumborhydrid bei Raumptemperatur, erwärmte anschließend auf 50 °C, fügte dann 10 ml konzentrierte Schwefelsäure hinzu, destillierte das Lösungsmittel zum größten Teil ab, schüttelte den Rückstand mit Wasser, saugte ab, wusch mit Wasser und trocknete. Dieses Produkt (33,1 g, Schmp. 154 °C) suspendierte man in 200 ml Methylenchlorid und fügte 0,1 ml Dimethylformamid und dann bei Raumtemperatur portionsweise 11 ml Thionylchlorid zu. Nach 24 Stunden destillierte man das Lösungsmittel ab, kochte den Rückstand mit 200 ml Methanol, kühlte auf 0 °C, saugte ab, wusch und trocknete. Man erhielt 30,1 g reines 6-(2,6-Dichlor-4-chlormethyl-phenoxymethyl)-4-methyl-2-pyron vom Schmp. 136 °C.

7,5 g der erhaltenen Verbindung rührte man mit 4 g 2-Thionaphthol, 30 ml Dimethylformamid und 7 g Kaliumcarbonat 24 Stunden bei Raumtemperatur, versetzte dann mit Wasser, nahm mit Methylenchlorid auf, wusch mit Wasser, trocknete und chromatographierte die Lösung über eine Säule mit Kieselgel. Man erhielt 8,5 g 6-[2,6-Dichlor-4-(naphth-2-yl-thiomethyl)-phenoxymethyl]-4-methyl-2-pyron vom Schmp. 125 °C. Dieses Produkt erwärmte man mit 25 g 2-Amino-pyridin auf 75 °C und gab innerhalb von 37 Stunden in 4 Portionen insgesamt 8 g Hydroxylamin-hydrochlorid zu. Nach 48 Stunden Reaktionsdauer nahm man mit Methylenchlorid auf, wusch mit verdünnter Salzsäure und Wasser, trocknete und destillierte das Lösungsmittel ab. Den Rückstand kristalliserte man einmal aus Acetonitril und einmal aus Äthylacetat um und erhielt so 2,1 g der reinen Verbindung 13 vom Schmp. 138 °C.

32 und 33: Analog der Arbeitsweise des Beispiels 31 wurde durch Umsetzung des Zwischenproduktes 6-(2,6-Dichlor-4-chlormethyl-phenoxymethyl)-4-methyl-2-pyron mit 4-Phenylphenol und Überführung des so erhaltenen Pyrons in das Hydroxypyridon die Verbindung 14 erhalten. Verwendung von 4-Hydroxy-benzaldehyd anstelle von 3,5-Dichlor-4-hydroxybenzaldehyd, sowie analoge Reuktion, Umsetzung mit Thionylchlorid, Kondensation mit 4-(4-Chlor-phenoxy)-phenol und Reaktion mit Hydroxylamin ergab die Verbindung 12.

34: 6-[4-(4-Chlor-phenoxy)-phenoxymethyl]-1-hydroxy-2-pyridon (Verbindung 22)

Man erhitzte 30 g 2-Brom-6-picolin mit 31,6 g N-Bromsuccinimid, 0,015 g Dibenzoylperoxyd und 150 ml Tetrachlorkohlenstoff unter UV-Bestrahlung 30 Stunden am Rückfluß, filtrierte, wusch das Filtrat einmal mit wäßriger Natriumcarbonat-Lösung und dreimal mit Wasser, trocknete und destillierte das Lösungsmittel unter vermindertem Druck ab. Den Rückstand (40,1 g) schüttelte man mit 150 ml Hexan und erhielt durch Absaugen 27,3 g eines Gemisches, das überwiegend aus der gewünschten Mono- neben etwas Dibrommethyl-Verbindung bestand. Dieses Gemisch rührte man zusammen mit 21,4 g 4-(4-Chlorphenoxy)-phenol, 20,7 g Kaliumcarbonat und 50 ml Dimethylformamid 48 Stunden bei Raumtemperatur, fügte dann 200 ml Methylenchlorid hinzu, wusch dreimal mit Wasser, engte die organische Lösung ein und isolierte durch Chromatographie über Kieselgel und Umkristallisieren aus Diisopropyläther 16,3 g 2-Brom-6-[4-(4-chlor-phenoxy)-phenoxymethyl]-pyridin.

15,8 g der erhaltenen Verbindung erwärmte man mit der Lösung von 8,5 g Peressigsäure in 50 ml Eisessig 30 Stunden auf 50 °C, destillierte dann das Lösungsmittel bei 40 °C unter vermindertem Druck teilweise ab, schüttelte den Rückstand dreimal mit je 200 ml Wasser und einmal mit wäßriger Natriumhydrogencarbonat-Lösung und dekantierte jeweils ab, behandelte schließlich mit 100 ml Diisopropyläther, saugte ab und trocknete. Man erhielt auf diese Weise 10,2 g fast reines N-Oxid vom Schmp. 100 °C. Man erwärmte 5 g dieses N-Oxids mit der Lösung von 1,2 g Natriumhydroxid in einer Mischung aus 9 ml Wasser und 20 ml Äthylenglykolmonomethyläther auf 70 °C. Dabei bildete sich durch Reaktion mit dem Alkohol rasch der bei 125 °C schmelzende Methoxyäthyläther des N-Oxids, der dann langsam verseift wurde. Nach 60 Stunden destillierte man das Lösungsmittel unter vermindertem Druck ab, schüttelte den Rückstand mit 200 ml Methylenchlorid und 50 ml verdünnter Schwefelsäure, trennte die organische Phase ab, trocknete sie und dampfte sie ein. Den Rückstand kristallisierte man aus Acetonitril um und erhielt 2,5 g der reinen Verbindung 22 vom Schmp. 180 °C.

35: 1-Hydroxy-4-methyl-6-[3-(naphth-1-yl-methoxy)-phenylthiomethyl]-2-pyridon

Man löste 26 g Monothioresorcin und 31,8 g 6-Chlormethyl-4-methyl-2-pyron in 100 ml Dimethylformamid, gab unter Rühren und Kühlen mit Eis innerhalb von 30 Minuten 38 g gepulvertes Kaliumcarbonat zu, rührte noch 4 Stunden bei 0 °C und 16 Stunden bei Raumtemperatur nach, fügte dann 300 ml Methylenchlorid zu, schüttelte dreimal mit Wasser aus, trennte, trocknete und destillierte das Lösungsmittel ab. Den Rückstand kristallierte man aus Methanol um und erhielt 44 g 6-(3-Hydroxy-phenylthiomethyl)-4-methyl-2-pyron Verbindung (C) vom Schmp. 129 °C. Zu einer Lösung von 8 g Natriumjodid in 200 ml Aceton gab man 8,9 g 1-Chlormethyl-naphthalin, rührte 16 Stunden bei Raumtemperatur, löste dann 12,4 g der Verbindung C in dem Ansatz auf, kühlte auf 0 °C und gab portionsweise innerhalb von 5 Stunden 6,9 g gepulvertes Kaliumcarbonat zu. Nach insgesamt 79 Stunden Reaktionszeit bei 0 °C destillierte man das Lösungsmittel im Vakuum der Wasserstrahlpumpe ab, nahm den Rückstand mit Methylenchlorid auf, wusch mit Wasser, trennte, trocknete, engte die Lösung ein, chromatographierte über eine Säule mit Kieselgel unter Verwendung von Methylenchlorid als Laufmittel und kristallisierte das Produkt der Hauptfraktionen aus Methanol um. Man erhielt 9 g reines 4-Methyl-6-[3-(naphth-1-yl-methoxy)-phenylthiomethyl]-2-pyron vom Schmp. 139 °C.

8,5 g dieser Verbindung erhitzte man mit 50 g 2-Aminopyridin auf 75 °C und fügte innerhalb von 40 Stunden portionsweise 8,9 g Hydroxylamin-hydrochlorid zu. Nach einer Reaktionszeit von 60 Stunden kühlte man auf Raumtemperatur, fügte Methylenchlorid zu, wusch einmal mit verdünnter Salzsäure und dreimal mit Wasser, trocknete und destillierte das Lösungsmittel ab. Den Rückstand kristallisierte man aus Äthylacetat um und erhielt 4 g Hydroxypyridon vom Schmp. 163 °C.

36: Setzte man das Pyron C (vgl. Beispiel 35) mit 4-Chlorbenzylchlorid um und verfuhr im übrigen wie im Beispiel 35, so erhielt man die Verbindung 42.

37: 1-Hydroxy-4-methyl-6-[4-(4-chlorphenoxy)-phenoxymethyl]-2-pyridon

171,4 g (0,5 Mol) 4-Methyl-6-[4-(4-chlorphenoxy)-phenoxymethyl]-2-pyron in 50 ml Toluol wurden auf 80 °C erwärmt. Dann wurden 59,9 g (0,36 Mol) Hydroxylammoniumsulfat und 38,3 g (0,36 Mol) Natriumcarbonat hinzugeben. 10 Minuten später wurden nochmals 59,9 g (0,36 Mol) Hydroxylammoniumsulfat und 38,3 g (0,36 Mol) Natriumcarbonat zugesetzt. Nach ca. 4 Stunden wurde die Heizung entfernt und bei etwa 40 °C 500 ml Methylenchlorid zugegeben. Das gelöste Reaktionsprodukt wurde dann von den ungelösten Salzen abfiltriert. Anschließend wurde das Filtrat über Natriumsulfat getrocknet und das Methylenchlorid abgedampft. Beim Verrühren des Rückstands mit 500 ml Ethylacetat kristallisierte das Reaktionsprodukt aus. Zur endgültigen Reinigung wurde das 1-Hydroxy-4-methyl-6-[4-(4-chlorphenoxy)-phenoxymethyl]-2-pyridon aus Dimethylformamid umkristallisiert. Ausbeute 80,5 g (45 %); Schmelzpunkt 168 - 170 °C.

38: 1-Hydroxy-4-methyl-6-[3-(phenoxyethoxy)-phenoxymethyl]-2-pyridon (Verbindung 45)

Man rührte eine Mischung von 80 g 6-Chlormethyl-4-methyl-2-pyron, 220 g Resorcin, 400 ml Dimethylformamid und 105 g fein gemahlenes Kaliumcarbonat 72 Stunden bei Raumtemperatur, fügte dann Methylenchlorid hinzu, schüttelte mehrmals mit Wasser aus, trocknete die organische Phase und destillierte das Lösungsmittel unter vermindertem Druck ab. Aus dem viskosen Rückstand (223 g) isolierte man durch Verreiben mit Wasser und anschließendes Umkristallisieren aus Methanol 53 g 6-(3-Hydroxyphenoxymethyl)-4-methyl-2-pyron (Verbindung D) vom Schmp. 145 ° C.

10 g der Verbindung D rührte man mit 11,2 g 1-Jod-2-phenoxyethan (hergestellt durch Umsetzung von 2-Phenoxyethanol mit $SOCl_2$ und anschließenden Austausch von Chlor gegen Jod mit Natriumjodid in Aceton), 6,9 g Kaliumcarbonat und 50 ml Dimethylformamid 35 Stunden bei 50 ° C, versetzte mit Methylenchlorid, wusch mehrmals mit Wasser, trocknete und chromatographierte die Lösung über Kieselgel. Als Hauptprodukt isolierte man 10,4 g 4-Methyl-6-[3-(phenoxyethoxy)-phenoxymethyl]-2-pyron vom Schmp. 95 ° C. 10 g dieses Pyrons erhitzte man mit 50 g 2-Aminopyridin 63 Stunden auf 75 ° C unter portionsweiser Zugabe von 8,5 g Hydroxylamin-hydrochlorid, nahm dann mit Methylenchlorid auf, schüttelte mit verdünnter Salzsäure aus (pH der wässrigen Phase 3 bis 4), trocknete, destillierte das Lösungsmittel ab und kristallisierte den Rückstand aus Acetonitrol. Man erhielt 4,3 g des reinen Hydroxypyridons vom Schmp. 148 ° C.

39 und 40: Auf die gleiche Weise wie in Beispiel 38 erhielt man ausgehend von dem Zwischenprodukt D und 1-(4-Chlorphenylthio)-4-jodbutan die Verbindung 48 sowie aus D und 2-(4-Chlorphenylthio)-2'-joddiethylether die Verbindung 49.

41: 6-[3-(4-Chlorphenylthio-propylthio)-phenoxymethyl]-1-hydroxy-4-methyl-2-pyridon (Verbindung 47)

Man rührte eine Mischung aus 12,6 g Monothioresorcin, 31,3 g 1-(4-Chlorphenylthio)-3-jod-propan (hergestellt aus 4-Chlorthiophenol und 1-Brom-3-chlorpropan und anschließendem Austausch von Chlor gegen Jod mit Natriumjodid in Aceton), 16,6 g Kaliumcarbonat und 60 ml Aceton 24 Stunden bei Raumtemperatur, destillierte dann das Lösungsmittel unter vermindertem Druck ab, fügte Methylenchlorid zu, wusch mehrmals mit Wasser, trocknete und isolierte dann durch Chromatographie über Kieselgel mit Methylenchlorid als Laufmittel 14,5 g 3-(4-Chlorphenylthio-propylthio)-phenol. Dieses Produkt rührte man zusammen mit 9,5 g 6-Chlormethyl-4-methyl-2-pyron, 10,4 g Kaliumcarbonat und 60 ml Aceton 31 Stunden bei 50 ° C, destillierte dann das Lösungsmittel unter vermindertem Druck ab, nahm den Rückstand mit Methylenchlorid auf, wusch mehrmals mit Wasser, trocknete und chromatographierte über Kieselgel. 11,2 g der Hauptfraktion erhitzte man mit 50 g 2-Aminopyridin 65 Stunden auf 75 ° C und fügte in mehreren Portionen insgesamt 12 g Hydroxylamin-hydrochlorid zu. Anschließend nahm man mit Methylenchlorid auf, schüttelte mit verdünnter Salzsäure und mehrmals mit Wasser aus, trocknete und destillierte das Lösungsmittel ab. Der Rückstand betrug 9,9 g. Beim Behandeln mit Methanol resultierten 2,7 g reines Hydroxypyridon vom Schmp. 102 ° C.

42 und 43: Bei gleichem Ablauf der Reaktionsfolge und unter gleichen Bedingungen erhielt man ausgehend von Monothioresorcin und 1-(4-Chlorphenylthio)-2,2-dimethyl-3-jod-propan die Verbindung 51 sowie aus Monothioresorcin und (4-Chlorphenylthio)-ethoxyethoxyethyljodid die Verbindung 53.

Untersuchung der Wirksamkeit

Bei der in vitro Untersuchung antimykotischer Substanzen muß zwischen einer Wirkung auf proliferierende (Fungistase) und ruhende Keime (Fungizidie) unterschieden werden.

Die Fungizidie, getestet am nicht wachsenden Pilz, wird als erschwertes Modell eingestuft. Hierzu wird in Mikrotitrationsplatten eine Verdünnungsreihe der zu testenden Präparate erstellt (31,25 bis 0,25 µg/ml; 8 Schritte). Jede U-förmige Vertiefung auf der Platte wird mit $10^4$ koloniebildenden Einheiten (CFU) des Hautpilzes Trichophyton mentagrophytes inokuliert (Milieu: Physiol. NaCl Lösung). Nach 18 h Inkubation bei 30 ° C werden die Keime mit 50 % Polyäthylenglykol 400 und NaCl-Lösung gewaschen (zweimalige Zentrifugation) und zur Keimzahlbestimmung auf Malz-Agarplatten mit Hilfe eines automatischen Gerätes ausgestrichen. Nach Bebrütung bei 30 ° C über 3 Tage werden die Kolonien gezählt und die CFU/ml berechnet. Im Vergleich mit der unbehandelten Kontrolle wird die Reduktion der Keimzahl in Prozent bestimmt (Kontrolle = 0 %). Die Wirkungsstärke wird an Standardpräparaten , z.B. Clotrimazol gemessen; Clotrimazol ist der "generic name" der Verbindung der Formel

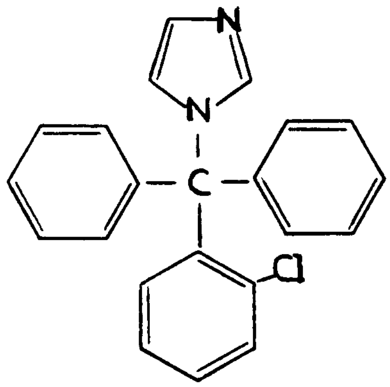

Wie aus Tabelle 1 ersichtlich, zeigten die erfindungsgemäßen Verbindungen eine äußerst niedrige Anzahl CFU im Verhältnis zum Standardpräparat Clotrimazol, d.h. der fungizide oder abtötende Effekt der erfindungsgemäßen Verbindungen ist deutlich stärker ausgeprägt als derjenige des Standardmittels.

Tabelle 1

| Präparat Nr. | Anzahl CFU/ml $\bar{x}$ (n = 4) | Reduktion CFU gegen Kontrolle in % |
|---|---|---|
| 1 | 0 | 100 |
| 9 | 0 | 100 |
| 15 | 1 | 99,32 |
| 17 | 1,5 | 98,98 |
| Clotrimazol | 63,6 | 57 |
| unbehandelte Kontrolle | 147,9 | 0 |
| n = Anzahl der Meßwerte<br>$\bar{x}$ = Mittelwert | | |

Als Beispiel für die hohe lokale in vivo Wirksamkeit der erfindungsgemäßen Verbindungen werden Behandlungsergebnisse an experimentell mit Trichophyton mentagrophytes infizierten Labortieren angeführt. Hierzu wurden jeweils zwei bzw. vier 450 - 500 g schwere Meerschweinchen (Stamm Pirbright white) mit 1,5 x $10^4$ Konidien/Tier in die Epidermis, verteilt auf jeweils 6 Infektionspunkte, infiziert. Die Behandlung der Tiere erfolgte 4 und 3 Tage vor der Infektion durch Aufbringen einer 0,3 %igen Präparatlösung auf jeweils 3 Infektionsstellen der rechten Rückenseite. Die linke Rückenseite mit jeweils 3 Infektionsstellen wurde mit Vehikel ohne Präparat auf gleiche Weise behandelt (Vehikelkontrolle).

Neben den mit den erfindungsgemäßen Substanzen behandelten Tieren wurden zwei Tiere mit der Referenzsubstanz Clotrimazol behandelt, und zwei infizierte Tiere blieben unbehandelt (Infektionskontrolle).

Wie aus Tabelle 2 ersichtlich, zeigten die erfindungsgemäßen Verbindungen eine deutlich größere Differenz der Mykosendurchmesser (mm) als das Standardpräparat Clotrimazol, d.h. der antimykotische Effekt der erfindungsgemäßen Verbindungen war demjenigen von Clotrimazol eindeutig überlegen.

Tabelle 2

Mykosen (Durchmesser in mm)

| Konzentration | Präparat Nr. | Anzahl Tiere | Vehikelkontrolle n | $\overline{x}_1$ | (s) | Präparat + Vehikel n | $\overline{x}_2$ | (s) | Differenz $\overline{x}_1 - \overline{x}_2$ (%) |
|---|---|---|---|---|---|---|---|---|---|
| Dermal | | | | | | | | | |
| 2 x 0,3 % | 1 | 4 | 12 | 14,8 | (2,7) | 12 | 9,2 | (3,4) | 5,6 (160,0) |
| | 3 | 4 | 12 | 13,7 | (2,2) | 12 | 8,3 | (1,2) | 5,4 (154,2) |
| | 9 | 2 | 6 | 14,0 | (3,4) | 6 | 9,1 | (1,8) | 4,9 (140,0) |
| | 26 | 2 | 6 | 15,0 | (1,5) | 6 | 7,8 | (0,4) | 7,2 (205,7) |
| | Clotrimazol | 4 | 12 | 13,9 | (2,0) | 12 | 10,4 | (1,8) | 3,5 (100,0) |
| Infektionskontrolle | - | 2 | 12 | 13,7 | (1,1) | | | | |

n = Anzahl Meßwerte

$\overline{x}$ = Mittelwert

(s)= Standardabweichung

## Patentansprüche

## Patentansprüche für folgende Vertragsstaaten : BE, CH, DE, FR, GB, IT, LI, NL, SE

**1.** 1-Hydroxy-2-pyridone der allgemeinen Formel I

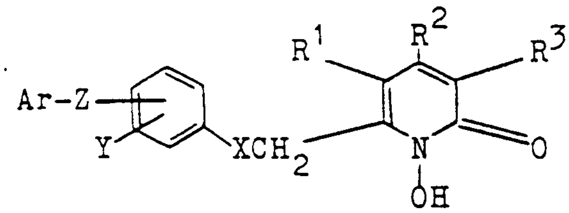

I,

worin bedeuten

$R^1$, $R^2$ und $R^3$, die gleich oder verschieden sind, Wasserstoff oder niederes Alkyl mit 1 - 4 Kohlenstoffatomen,

X S oder O,

Y Wasserstoff oder bis zu 2 Halogenatome, nämlich Chlor und/oder Brom,

Z eine Einfachbindung oder die zweiwertigen Reste O, S, $-CR_2-$ (R = H oder $C_1$ - $C_4$-Alkyl) oder andere 2-wertige Reste mit 2 - 10 kettenförmig verknüpften C- und gegebenenfalls O- und/oder S-Atomen, wobei - wenn die Reste 2 oder mehr O- und/oder S-Atome enthalten - letztere durch mindestens 2 C-Atome voneinander getrennt sein müssen und wobei 2 benachbarte C-Atome auch durch eine Doppelbindung minteinander verknüpft sein können und die freien Valenzen der C-Atome durch H und/oder $C_1$-$C_4$ -Alkylgruppen abgesättigt sind,

Ar ein aromatisches Ringsystem mit bis zu zwei Ringen, ausgewählt aus der Gruppe Phenyl, Naphthyl, Tetrahydronaphthyl, Indenyl, Biphenyl, Diphenylalkan, Diphenylether und Diphenylthioether,
das durch bis zu drei Reste aus der Gruppe Fluor, Chlor, Brom, Methoxy, $C_1$-$C_4$ -Alkyl, Trifluormethyl und Trifluormethoxy substituiert sein kann.

2. Verbindungen nach Anspruch 1, dadurch gekennzeichnet, daß Ar den - gegebenenfalls substituierten - Phenylring darstellt.

3. Verbindungen nach Anspruch 1, dadurch gekennzeichnet, daß Ar ein isoliertes bicyclisches System darstellt, das sich vorzugsweise vom Biphenyl, Diphenylalkan oder Diphenyläther ableitet und gegebenenfalls substituiert ist.

4. Verbindungen nach einem oder mehreren der Ansprüche 1 bis 3, dadurch gekennzeichnet, daß Z eine Einfachbindung ist.

5. Verbindungen nach einem oder mehreren der Ansprüche 1 bis 3, dadurch gekennzeichnet, daß Z Sauerstoff darstellt oder enthält.

6. Verbindungen nach einem oder mehreren der Ansprüche 1 bis 3, dadurch gekennzeichnet, daß $R^1$ und $R^3$ Wasserstoff sind $R^2$ Methyl ist und X Sauerstoff ist.

7. 6-[4-(4-Chlor-phenoxy)-phenoxymethyl]-1-hydroxy-4-methyl-2-pyridon (= Verbindung der Formel I gemäß der Definition in Anspruch 1 mit $R^1$ = $R^3$ = Y = H, $R^2$ = $CH_3$ , X = O, Z = 0 in 4-Stellung zur $XCH_2$-Gruppe, und

Ar = 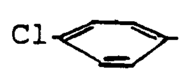 ).

8. 6-(Biphenylyl-4-oxy-methyl)-1-hydroxy-4-methyl-2-pyridon (= Verbindungen der Formel I gemäß der Definition in Anspruch 1 mit $R^1$ = $R^3$ = Y = H, $R^2$ = $CH_3$ , X = O, Z = Einfachbindung und Ar = $C_6H_5$ in 4-Stellung zur $XCH_2$-Gruppe).

9. 1-Hydroxy-4-methyl-6-[4-(4-trifluormethyl-phenoxy)-phenoxymethyl]-2-pyridon (= Verbindung der Formel I gemäß der Definition in Anspruch 1 mit $R^1$ = $R^3$ = Y = H, $R^2$ = $CH_3$ ,X = O, Z = O in 4-Stellung zur $XCH_2$-Gruppe, und

Ar = 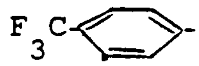 ).

**10.** Verfahren zur Herstellung von Verbindungen der Formel I gemäß einem oder mehreren der Ansprüche 1 bis 9, dadurch gekennzeichnet, daß man

a) ein 6-Halogenmethyl-2-pyron der Formel III

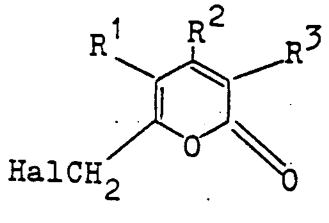

III

mit einem Phenol oder Thiophenol der Formel IV

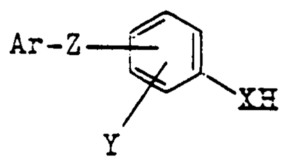

IV

umsetzt und die so gebildeten Aryloxymethyl-pyrone bzw. Arylthiomethyl-pyrone der Formel V

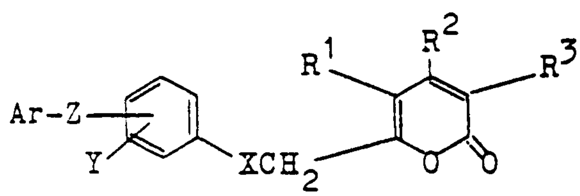

V

durch Einwirkung von Hydroxylamin in die Hydroxypyridone der Formel I umwandelt, oder daß man

b) ein Dihalogenpicolin der Formel VI

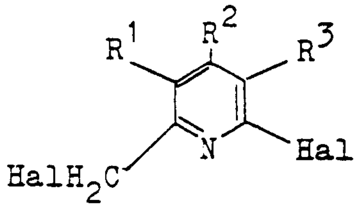

VI

mit einem Phenol der Formel IV umsetzt, die erhaltene Verbindung zum N-Oxid oxidiert und die Verbindung durch Verseifung des kernständigen Halogens in eine Verbindung gemäß Formel I überführt,

wobei in den Formeln I und III bis VI die Reste $R^1$ ,$R^2$,$R^3$, X, Y, Z und Ar jeweils die in den Ansprüchen 1 bis 9 angegebene Bedeutung haben und Hal ein Halogenatom, insbesondere Chlor oder Brom darstellt, oder daß man in Verbindungen, die nach der Arbeitsweise a) oder b) erhalten wurden, oder in solche Zwischenprodukte, die in Ar oder dem Pyridonring noch reaktionsfähige Gruppen enthalten, durch deren Austausch noch andere Substituenten einführt, die den Definitionen der Reste $R^1$, $R^2$, $R^3$

und der Substituenten von Ar genügen.

**11.** Verfahren nach Anspruch 10, dadurch gekennzeichnet, daß man in Alternative a die Umwandlung der Aryloxymethylpyrone bzw. Arylthiomethylpyrone der Formel V in die Hydroxypyridone der Formel I durch Umsetzung mit mindestens etwa 1 Mol eines Hydroxylammoniumsalzes pro Mol Verbindung der Formel V in Gegenwart von mindestens etwa einem Äquivalent wenigstens einer organischen oder anorganischen Base, bezogen auf das Hydroxylammoniumsalz, durchführt.

**12.** Verfahren nach Anspruch 11, dadurch gekennzeichnet, daß man als Basen die Carbonate und/oder Hydrogencarbonate der Alkalimetalle, vorzugsweise von Natrium und/oder Kalium, insbesondere $Na_2CO_3$, verwendet.

**13.** Verfahren nach Anspruch 12, dadurch gekennzeichnet, daß man als Hydroxylammoniumsalz Hydroxylammoniumsulfat verwendet.

**14.** Verbindungen der Formel I gemäß einem oder mehreren der Ansprüche 1 bis 9 oder wie erhalten nach einem oder mehreren der Ansprüche 10 bis 13, und/oder mindestens ein physiologisch verträgliches Salz einer solchen Verbindung mit einer anorganischen oder organischen Base zur Anwendung als Heilmittel.

**15.** Arzneimittel, enthaltend eine wirksame Menge mindestens einer Verbindung der Formel I gemäß einem oder mehreren der Ansprüche 1 bis 9 oder wie erhalten nach einem oder mehreren der Ansprüche 10 bis 13, und/oder mindestens eines physiologisch verträglichen Salzes einer solchen Verbindung mit einer anorganischen oder organischen Base, neben einem physiologisch annehmbaren Träger und gegebenenfalls weiteren Zusatz-und/oder Hilfsstoffen.

**16.** Arzneimittel gemäß Anspruch 15 zur Verwendung als Antimykotikum, insbesondere gegen pathogene Haut- und Schleimhautpilze.

**17.** Verfahren zur Herstellung eines Arzneimittels gemäß Anspruch 15 oder 16, dadurch gekennzeichnet, daß man mindestens eine Verbindung der Formel I gemäß einem oder mehreren der Ansprüche 1 bis 9 oder wie erhalten nach einem oder mehreren der Ansprüche 10 bis 13 und/oder mindestens ein physiologisch verträgliches Salz einer solchen Verbindung mit einer anorganischen oder organischen Base mit einem physiologisch annehmbaren Träger und gegebenenfalls weiteren Zusatz- und/oder Hilfsstoffen in eine geeignete Darreichungsform bringt.

**18.** Verbindungen der Formel V

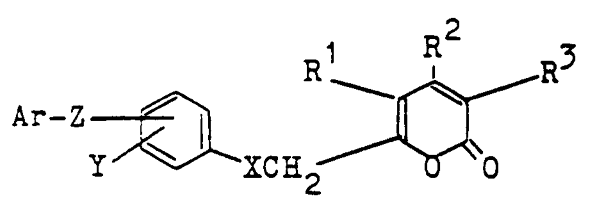

V

worin $R^1$, $R^2$, $R^3$, X, Y und Ar die in den Ansprüchen 1 bis 9 genannte Bedeutung haben.

**19.** Verfahren zur Herstellung der Verbindungen der Formel V gemäß Anspruch 18, dadurch gekennzeichnet, daß man ein 6-Halogenmethyl-2-pyron der Formel III

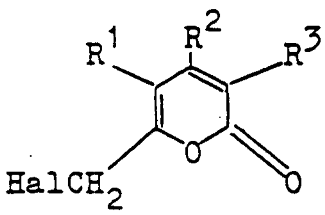

III

mit einem Phenol oder Thiophenol der Formel IV

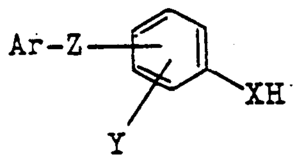

umsetzt, wobei in den Formeln die Reste $R_1$, $R_2$, $R_3$, X, Y, Z und Ar die in den Ansprüchen 1 bis 9 genannte Bedeutung haben und
Hal ein Halogenatom, insbesondere Chlor oder Brom, darstellt.

**Patentansprüche für folgende Vertragsstaaten : AT, ES, GR**

**1.** Verfahren zur Herstellung von Verbindungen der Formel I

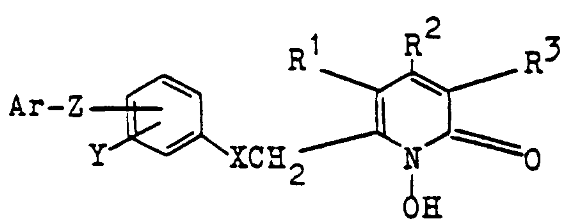

I,

worin bedeuten

| | |
|---|---|
| $R^1$, $R^2$ und $R^3$, | die gleich oder verschieden sind, Wasserstoff oder niederes Alkyl mit 1 - 4 Kohlenstoffatomen, |
| X | S oder O, |
| Y | Wasserstoff oder bis zu 2 Halogenatome, nämlich Chlor und/oder Brom, |
| Z | eine Einfachbindung oder die zweiwertigen Reste O, S, $-CR_2-$ (R = H oder $C_1$-$C_4$-Alkyl) oder andere 2-wertige Reste mit 2 - 10 kettenförmig verknüpften C- und gegebenenfalls O- und/oder S-Atomen, wobei - wenn die Reste 2 oder mehr O- und/oder S-Atome enthalten - letztere durch mindestens 2 C-Atome voneinander getrennt sein müssen und wobei 2 benachbarte C-Atome auch durch eine Doppelbindung minteinander verknüpft sein können und die freien Valenzen der C-Atome durch H und/oder $C_1$-$C_4$-Alkylgruppen abgesättigt sind, |
| Ar | ein aromatisches Ringsystem mit bis zu zwei Ringen, ausgewählt aus der Gruppe Phenyl, Naphthyl, Tetrahydronaphthyl, Indenyl, Biphenyl, Diphenylalkan, Diphenylether und Diphenylthioether, das durch bis zu drei Reste aus der Gruppe Fluor, Chlor, Brom, Methoxy, $C_1$-$C_4$-Alkyl, Trifluormethyl und Trifluormethoxy substituiert sein kann, |

dadurch gekennzeichnet, daß man

a) ein 6-Halogenmethyl-2-pyron der Formel III

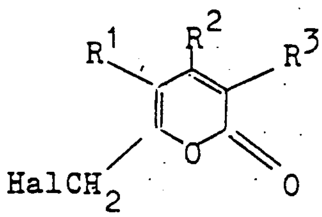

III

mit einem Phenol oder Thiophenol der Formel IV

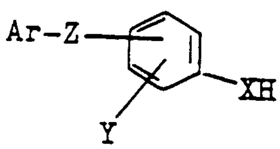

IV

umsetzt und die so gebildeten Aryloxymethyl-pyrone bzw. Arylthiomethyl-pyrone der Formel V

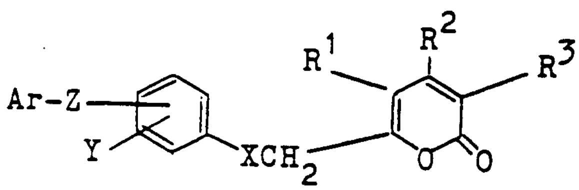

V

durch Einwirkung von Hydroxylamin in die Hydroxypyridone der Formel I umwandelt, oder daß man b) ein Dihalogenpicolin der Formel VI

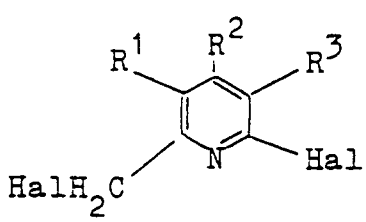

VI

mit einem Phenol der Formel IV umsetzt, die erhaltene Verbindung zum N-Oxid oxidiert und die Verbindung durch Verseifung des kernständigen Halogens in eine Verbindung gemäß Formel I überführt,

wobei in den Formeln I und III bis VI die Reste $R^1$, $R^2$, $R^3$, X, Y, Z und Ar die gleiche Bedeutung wie in Formel I haben und Hal ein Halogenatom, insbesondere Chlor oder Brom darstellt, oder daß man in Verbindungen, die nach der Arbeitsweise a) oder b) erhalten wurden, oder in solche Zwischenprodukte, die in Ar oder dem Pyridonring noch reaktionsfähige Gruppen enthalten, durch deren Austausch noch andere Substituenten einführt, die den Definitionen der Reste $R^1$, $R^2$, $R^3$ und der Substituenten von Ar genügen.

**2.** Verfahren nach Anspruch 1, dadurch gekennzeichnet, daß man solche Verbindungen der Formel I herstellt, worin Ar den - gegebenenfalls substituierten - Phenylring darstellt.

**3.** Verfahren nach Anspruch 1, dadurch gekennzeichnet, daß man solche Verbindungen der Formel I herstellt, worin Ar ein isoliertes bicyclisches System darstellt, das sich vorzugsweise vom Biphenyl, Diphenylalkan oder Diphenyläther ableitet und gegebenenfalls substituiert ist.

**4.** Verfahren nach einem oder mehreren der Ansprüche 1 bis 3, dadurch gekennzeichnet, daß man solche

Verbindungen der Formel I herstellt, worin Z eine Einfachbindung ist.

**5.** Verfahren nach einem oder mehreren der Ansprüche 1 bis 3, dadurch gekennzeichnet, daß man solche Verbindungen der Formel I herstellt, worin Z Sauerstoff darstellt oder enthält.

**6.** Verfahren nach einem oder mehreren der Ansprüche 1 bis 3, dadurch gekennzeichnet, daß man solche Verbindungen der Formel I herstellt, worin $R^1$ und $R^3$ Wasserstoff sind, $R^2$ Methyl ist und X Sauerstoff ist.

**7.** Verfahren nach Anspruch 1, dadurch gekennzeichnet, daß man 6-[4-(4-Chlor-phenoxy)-phenoxymethyl]-1-hydroxy-4-methyl-2-pyridon (= Verbindung der Formel I gemäß der Definition in Anspruch 1 mit $R^1 = R^3 = Y = H$, $R^2 = CH_3$, X = O, Z = O in 4-Stellung zur $XCH_2$-Gruppe, und

Ar = 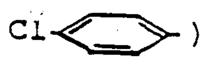

herstellt.

**8.** Verfahren nach Anspruch 1, dadurch gekennzeichnet, daß man 6-(Biphenylyl-4-oxy-methyl)-1-hydroxy-4-methyl-2-pyridon (= Verbindungen der Formel I gemäß der Definition in Anspruch 1 mit $R^1 = R^3 = Y = H$, $R^2 = CH_3$, X = O, Z = Einfachbindung und Ar = $C_6H_5$ in 4-Stellung zur $XCH_2$-Gruppe) herstellt.

**9.** Verfahren nach Anspruch 1, dadurch gekennzeichnet, daß man 1-Hydroxy-4-methyl-6-[4-(4-trifluormethyl-phenoxy)-phenoxymethyl]-2-pyridon (= Verbindung der Formel I gemäß der Definition in Anspruch 1 mit $R^1 = R^3 = Y = H$, $R^2 = CH_3$, X = O, Z = O in 4-Stellung zur $XCH_2$-Gruppe, und

Ar = 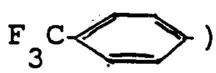

herstellt.

**10.** Verfahren nach einem oder mehreren der Ansprüche 1 bis 9, dadurch gekennzeichnet, daß man in Alternative a die Umwandlung der Aryloxymethylpyrone bzw. Arylthiomethylpyrone der Formel V in die Hydroxypyridone der Formel I durch Umsetzung mit mindestens etwa 1 Mol eines Hydroxylammoniumsalzes pro Mol Verbindung der Formel V in Gegenwart von mindestens etwa einem Äquivalent wenigstens einer organischen oder anorganischen Base, bezogen auf das Hydroxylammoniumsalz, durchführt.

**11.** Verfahren nach Anspruch 10, dadurch gekennzeichnet, daß man als Basen die Carbonate und/oder Hydrogencarbonate der Alkalimetalle, vorzugsweise von Natrium und/oder Kalium, insbesondere $Na_2CO_3$, verwendet.

**12.** Verfahren nach Anspruch 11, dadurch gekennzeichnet, daß man als Hydroxylammoniumsalz Hydroxylammoniumsulfat verwendet.

**13.** Verfahren zur Herstellung eines Arzneimittels, dadurch gekennzeichnet, daß man mindestens eine Verbindung der Formel I wie erhalten nach einem oder mehreren der Ansprüche 1 bis 12 und/oder mindestens ein physiologisch verträgliches Salz einer solchen Verbindung mit einer anorganischen oder organischen Base
mit einem physiologisch annehmbaren Träger und gegebenenfalls weiteren Zusatz- und/oder Hilfsstoffen in eine geeignete Darreichungsform bringt.

**14.** Verbindungen der Formel V

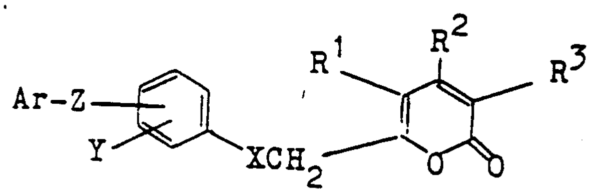

V

worin $R^1$, $R^2$, $R^3$, X, Y, Z und Ar die in den Ansprüchen 1 bis 9 genannte Bedeutung haben.

**15.** Verfahren zur Herstellung der Verbindungen der Formel V gemäß Anspruch 14, dadurch gekennzeichnet, daß man ein 6-Halogenmethyl-2-pyron der Formel III

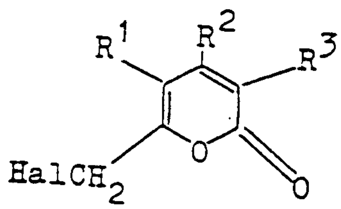

III

mit einem Phenol oder Thiophenol der Formel IV

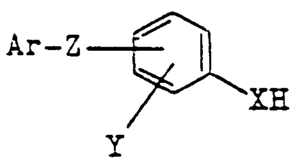

IV

umsetzt, wobei in den Formeln die Reste $R_1$, $R_2$, $R_3$, X, Y, Z und Ar die in den Ansprüchen 1 bis 9 genannte Bedeutung haben und
Hal ein Halogenatom, insbesondere Chlor oder Brom, darstellt.

**Claims**
**Claims for the following Contracting States : BE, CH, DE, FR, GB, IT, LI, NL, SE**

**1.** A 1-hydroxy-2-pyridone of the formula I

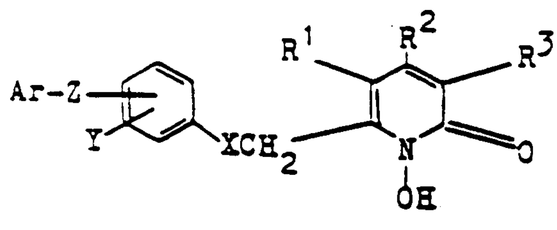

I,

in which

| | |
|---|---|
| $R^1$, $R^2$ and $R^3$, | which are identical or different, denote hydrogen or lower alkyl having 1 - 4 carbon atoms, |
| X | denotes S or O, |
| Y | denotes hydrogen or up to 2 halogen atoms, namely chlorine and/or bromine, |
| Z | denotes a single bond or the bivalent radicals O, S, $-CR_2-$ (R = H or $C_1$-$C_4$-alkyl) or other 2-valent radicals with 2 - 10 carbon and, optionally, oxygen and/or sulfur atoms linked to form a chain, it being obligatory when the radicals contain 2 or more oxygen and/or sulfur atoms for the latter to be separated by at least 2 |

carbon atoms, and it being possible for 2 adjacent carbon atoms also to be linked together by a double bend, and the free valencies of the carbon atoms being saturated by H and/or $C_1$-$C_4$-alkyl groups,

Ar denotes an aromatic ring system which has up to two rings and is selected from the group comprising phenyl, naphthyl, tetrahydronaphthyl, indenyl, biphenyl, diphenylalkane, diphenyl ether and diphenyl thioether, and which can be substituted by up to three radicals from the group comprising fluorine, chlorine, bromine, methoxy, $C_1$-$C_4$-alkyl, trifluoromethyl and trifluoromethoxy.

**2.** A compound as claimed in claim 1, wherein Ar represents the phenyl ring, which is optionally substituted.

**3.** A compound as claimed in claim 1, wherein Ar represents an isolated bicyclic system which is preferably derived from biphenyl, diphenylalkane or diphenyl ether and is optionally substituted.

**4.** A compound as claimed in one or more of claims 1 to 3, wherein Z is a single bond.

**5.** A compound as claimed in one or more of claims 1 to 3, wherein Z represents or contains oxygen.

**6.** A compound as claimed in one or more of claims 1 to 3, wherein $R^1$ and $R^3$ are hydrogen, $R^2$ is methyl and X is oxygen.

**7.** 6-[4-(4-Chlorophenoxy)phenoxymethyl]-1-hydroxy-4-methyl-2-pyridone (= compound of the formula I as defined in claim 1 with $R^1$ = $R^3$ = Y = H, $R^2$ = $CH_3$, X = O, Z = O in the 4-position to the $XCH_2$ group, and

Ar = 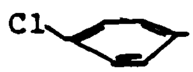 ).

**8.** 6-(4-Biphenylyloxymethyl)-1-hydroxy-4-methyl-2-pyridone (= compound of the formula I as defined in claim 1 with $R^1$ = $R^3$ = Y = H, $R^2$ = $CH_3$, X = O, Z = single bond, and Ar - $C_6H_5$ in the 4-position to the $XCH_2$ group).

**9.** 1-Hydroxy-4-methyl-6-[4-(4-trifluoromethylphenoxy)-phenoxymethyl]-2-pyridone (= compound of the formula I as defined in claim 1 with $R_1$ = $R^3$ = Y = H, $R^2$ = $CH_3$, X = O Z = O in the 4-position to the $XCH_2$ group, and

Ar = 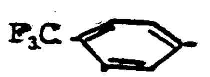 ).

**10.** A process for the preparation of a compound of the formula I as claimed in one or more of claims 1 to 9, which comprises

a) reaction of a 6-halogenomethyl-2-pyrone of the formula III

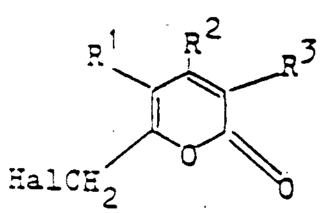

III

with a phenol or thiophenol of the formula IV

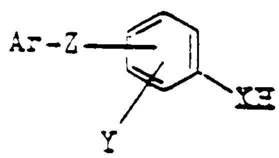

IV

and conversion of the aryloxymethylpyrone or arylthiomethylpyrone of the formula V which has been formed

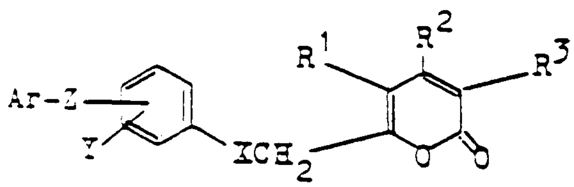

V

by the action of hydroxylamine into the hydroxypyridone of the formula I, or comprises
b) reaction of a dihalogenopicoline of the formula VI

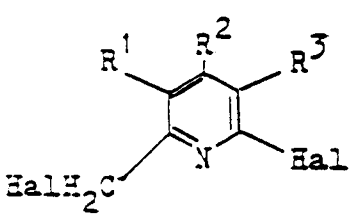

VI

with a phenol of the formula IV, oxidation of the resulting compound to give the N-oxide, and conversion of the compound into a compound of the formula I by hydrolysis of the halogen on the nucleus, the radicals $R^1$, $R^2$, $R^3$, X, Y, Z and Ar in the formulae I and III to VI each having the meaning indicated in claims 1 to 9, and Hal representing a halogen atom, in particular chlorine or bromine, or comprises introduction of other substituents, which comply with the definitions of the radicals $R^1$, $R^2$, $R^3$ and of the substituents of Ar, into compounds which have been obtained by procedure a) or b), or into those intermediates which still contain reactive groups in Ar or the pyridone ring, by replacement thereof.

**11.** The process as claimed in claim 10, wherein, in alternative a, the conversion of the aryloxymethylpyrone or arylthiomethylpyrone of the formula V into the hydroxypyridone of the formula I is carried out by reaction with at least about 1 mole of a hydroxylammonium salt per mole of compound of the formula V, in the presence of at least about one equivalent of at least one organic or inorganic base, relative to the hydroxylammonium salt.

**12.** The process as claimed in claim 11, wherein the bases used are the carbonates and/or bicarbonates of the alkali metals, preferably of sodium and/or potassium, in particular $Na_2CO_3$.

**13.** The process as claimed in claim 12, wherein the hydroxylammonium salt used is hydroxylammonium sulfate.

**14.** A compound of the formula I as claimed in one or more of claims 1 to 9 or as obtained as claimed in one or more of claims 10 to 13, and/or at least one physiologically tolerated salt of a compound of this type with an inorganic or organic base, for use as a medicine.

**15.** A medicament containing an effective amount of at least one compound of the formula I as claimed in one or more of claims 1 to 9 or as obtained as claimed in one or more of claims 10 to 13, and/or at least one physiologically tolerated salt of a compound of this type with an inorganic or organic base, in addition to a physiologically acceptable vehicle and, where appropriate, further additives and/or auxiliaries.

**16.** A medicament as claimed in claim 15 for use as an antimycotic, in particular against pathogenic skin fungi and mucous membrane fungi.

**17.** A process for the preparation of a medicament as claimed in claim 15 or 16, which comprises conversion into a suitable presentation of at least one compound of the formula I as claimed in one or more of claims 1 to 9 or as obtained as claimed in one or more of claims 10 to 13, and/or at least one physiologically tolerated salt of a compound of this type with an inorganic or organic base, with a physiologically acceptable vehicle and, where appropriate, further additives and/or auxiliaries.

**18.** A compound of the formula V

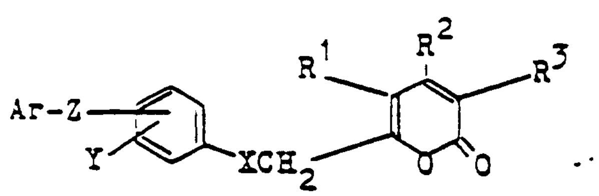

V

in, which $R^1$, $R^2$, $R^3$, X, Y, Z and Ar have the meaning stated in claims 1 to 9.

**19.** A process for the preparation of a compound of the formula V as claimed in claim 18, which comprises reaction of a 6-halogenomethyl-2-pyrone of the formula III

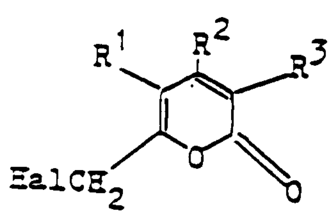

III

with a phenol or thiophenol of the formula IV

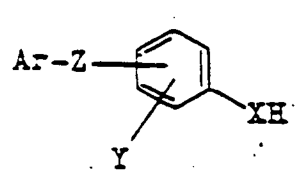

the radicals $R^1$, $R^2$, $R^3$, X, Y, Z and Ar in the formulae having the meanings stated in claims 1 to 9, and Hal representing a halogen atom, in particular chlorine or bromine.

**Claims for the following Contracting States : AT, ES, GR**

**1.** A process for the preparation of a compound of the formula I

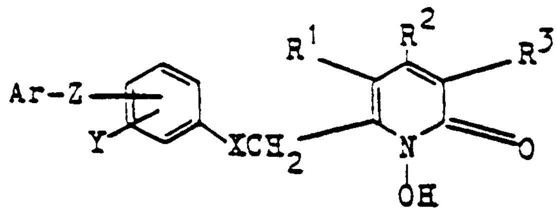

I,

in which

| | |
|---|---|
| $R^1$, $R^2$ and $R^3$, | which are identical or different, denote hydrogen or lower alkyl having 1 - 4 carbon atoms, |
| X | denotes S or O, |
| Y | denotes hydrogen or up to 2 halogen atoms, namely chlorine and/or bromine, |
| Z | denotes a single bond or the bivalent radicals O, S, $-CR_2-$ (R = H or $C_1$-$C_4$-alkyl) or other 2-valent radicals with 2 - 10 carbon and, optionally, oxygen and/or sulfur atoms linked to form a chain, it being obligatory when the radicals contain 2 or more oxygen and/or sulfur atoms for the latter to be separated by at least 2 carbon atoms, and it being possible for 2 adjacent carbon atoms also to be linked together by a double bond, and the free valencies of the carbon atoms being saturated by H and/or $C_1$-$C_4$-alkyl groups, |
| Ar | denotes an aromatic ring system which has up to two rings and is selected from the group comprising phenyl, naphthyl, tetrahydronaphthyl, indenyl, biphenyl, diphenylalkane, diphenyl ether and diphenyl thioether, and which can be substituted by up to three radicals from the group comprising fluorine, chlorine, bromine, methoxy, $C_1$-$C_4$-alkyl, trifluoromethyl and trifluoromethoxy, |

which comprises

a) reaction of a 6-halogenomethyl-2-pyrone of the formula III

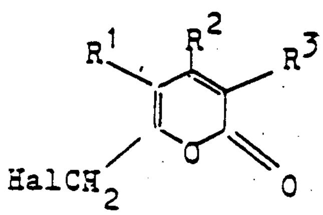

III

with a phenol or thiophenol of the formula IV

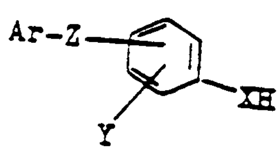

IV

and conversion of the aryloxymethylpyrone or arylthiomethylpyrone of the formula V which has been formed

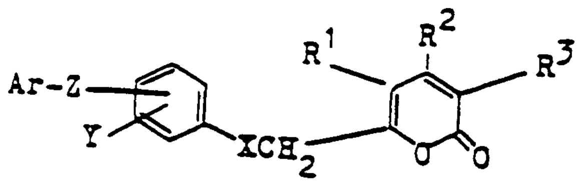

V

by the action of hydroxylamine into the hydroxypyridone of the formula I, or comprises
b) reaction of a dihalogenopicoline of the formula VI

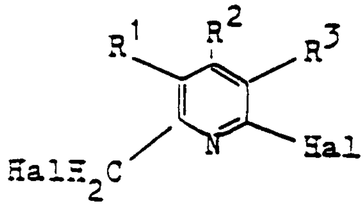

VI

with a phenol of the formula IV, oxidation of the resulting compound to give the N-oxide, and conversion of the compound into a compound of the formula I by hydrolysis of the halogen on the nucleus, the radicals $R^1$, $R^2$, $R^3$, X, Y, Z and Ar in the formulae I and III to VI each have the same meaning as in formula 1, and Hal representing a halogen atom, in particular chlorine or bromine, or comprises introduction of other substituents, which comply with the definitions of the radicals $R^1$, $R^2$, $R^3$ and of the substituents of Ar, into compounds which have been obtained by procedures a) or b), or into those intermediates which still contain reactive groups in Ar or the pyridone ring, by replacement thereof.

2. The process as claimed in claim 1, in which is prepared a compound of the formula I in which Ar represents the phenyl ring, which is optionally substituted.

3. The process as claimed in claim 1, in which is prepared a compound of the formula I in which Ar represents an isolated bicyclic system which is preferably derived from biphenyl, diphenylalkane or diphenyl ether and is optionally substituted.

4. The process as claimed in one or more of claims 1 to 3, in which is prepared a compound of the formula I in which Z is a single bond.

5. The process as claimed in one or more of claims 1 to 3, in which is prepared a compound of the formula I in which Z represents or contains oxygen.

6. The process as claimed in one or more of claims 1 to 3, in which is prepared a compound of the formula I in which $R^1$ and $R^3$ are hydrogen, $R^2$ is methyl and X is oxygen.

7. The process as claimed in claim 1, in which is prepared 6-[4-(4-chlorophenoxy)phenoxymethyl]-1-hydroxy-4-methyl-2-pyridone (= compound of the formula I as defined in claim 1 with $R^1 = R^3 = Y = H$, $R^2 = CH_3$, X = O, Z = O in the 4-position to the $XCH_2$ group, and

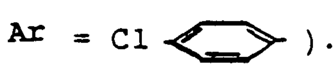

8. The process as claimed in claim 1, in which is prepared 6-(4-biphenylyloxymethyl)-1-hydroxy-4-methyl-2-pyridone (= compound of the formula I as defined in claim 1 with $R^1 = R^3 = Y = H$, $R^2 = CH_3$, X = O, Z = single bond, and Ar = $C_6H_5$ in the 4-position to the $XCH_2$ group).

**9.** The process as claimed in claim 1, in which is prepared 1-hydroxy-4-methyl-6-[4-(4-trifluoromethylphenoxy)-phenoxymethyl]-2-pyridone (= compound of the formula I as defined in claim 1 with $R_1 = R^3 = Y = H$, $R^2 = CH_3$, $X = O$, $Z = O$ in the 4-position to the $XCH_2$ group, and

Ar = 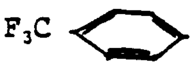 ).

**10.** The process as claimed in one or more of claims 1 to 9, wherein, in alternative a, the conversion of the aryloxymethylpyrone or arylthiomethylpyrone of the formula V into the hydroxypyridone of the formula I is carried out by reaction with at least about 1 mole of hydroxylammonium salt per mole of compound of the formula V, in the presence of at least about one equivalent of at least one organic or inorganic base, relative to the hydroxylammonium salt.

**11.** The process as claimed in claim 10, wherein the bases used are the carbonates and/or bicarbonates of the alkali metals, preferably of sodium and/or potassium, in particular $Na_2CO_3$.

**12.** The process as claimed in claim 11, wherein the hydroxylammonium salt used is hydroxylammonium sulfate.

**13.** A process for the preparation of a medicament, which comprises conversion into a suitable presentation of at least one compound of the formula I as obtained as claimed in one or more of claims 1 to 12, and/or at least one physiologically tolerated salt of a compound of this type with an inorganic or organic base, with a physiologically acceptable vehicle and, where appropriate, further additives and/or auxiliaries.

**14.** A compound of the formula V

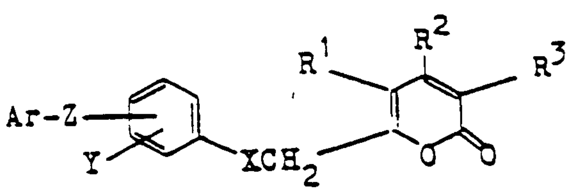

V

in which $R^1$, $R^2$, $R^3$, X, Y, Z and Ar have the meaning stated in claims 1 to 9.

**15.** A process for the preparation of a compound of the formula V as claimed in claim 14, which comprises reaction of a 6-halogenomethyl-2-pyrone of the formula III

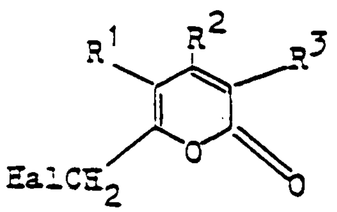

III

with a phenol or thiophenol of the formula IV

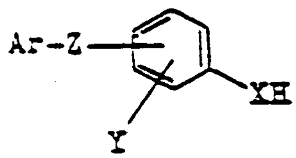

IV

the radicals $R^1$, $R^2$, $R^3$, X, Y, Z and Ar in the formulae having the meanings stated in claims 1 to 9, and Hal representing a halogen atom, in the particular chlorine or bromine.

**Revendications**

**Revendications pour les Etats contractants suivants : BE, CH, DE, FR, GB, IT, LI, NL, SE**

1. 1-hydroxy-2-pyridones de formule générale I

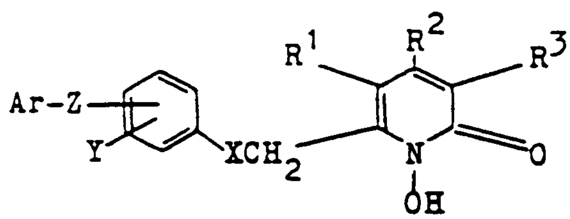

I

dans laquelle

| | |
|---|---|
| $R^1$, $R^2$ et $R^3$, | qui sont identiques ou différents, représentent un atome d'hydrogène ou un groupe alkyle inférieur ayant de 1 à 4 atomes de carbone, |
| X | représente S ou O, |
| Y | représente un atome d'hydrogène ou jusqu'à 2 atomes d'halogène, à savoir de chlore et/ou de brome, |
| Z | représente une simple liaison ou les radicaux bivalents O, S, $-CR_2-$ (R = H ou un groupe alkyle en $C_1$-$C_4$) ou d'autres radicaux bivalents ayant 2-10 atomes de carbone et éventuellement d'oxygène et/ou de soufre liés en formant une chaîne, lorsque les radicaux contiennent 2 atomes d'oxygène et/ou de soufre, ou plus, ces derniers devant être séparés l'un de l'autre (les uns des autres) par au moins 2 atomes de carbone, et 2 atomes de carbone contigus pouvant également être liés l'un à l'autre par une double liaison et les valences libres des atomes de carbone étant saturées par H et/ou des groupes alkyle en $C_1$-$C_4$, |
| Ar | représente un système cyclique aromatique comportant jusqu'à deux cycles, choisi parmi les systèmes phényle, naphtyle, tétrahydronaphtyle, indényle, biphényle, diphénylalcane, éther diphénylique et diphénylthioéther, qui peut être substitué par jusqu'à trois substituants choisis parmi des atomes de fluor, chlore et brome et des groupes méthoxy, alkyle en $C_1$-$C_4$, trifluorométhyle et trifluorométhoxy. |

2. Composés selon la revendication 1, caractérisés en ce que Ar représente le cycle phényle, éventuellement substitué.

3. Composés selon la revendication 1, caractérisés en ce que Ar représente un système bicyclique isolé qui dérive de préférence du biphényle, d'un diphénylalcane ou de l'éther diphénylique et éventuellement est substitué.

4. Composés selon une ou plusieurs des revendications 1 à 3, caractérisés en ce que Z est une simple liaison.

5. Composés selon une ou plusieurs des revendications 1 à 3, caractérisés en ce que Z représente ou contient un atome d'oxygène.

**6.** Composés selon une ou plusieurs des revendications 1 à 3, caractérisés en ce que $R^1$ et $R^3$ sont des atomes d'hydrogène, $R^2$ est le groupe méthyle et X est un atome d'oxygène.

**7.** 6-[4-(4-chlorophénoxy)-phénoxyméthyl]-1-hydroxy-4-méthyl-2-pyridone (= composé de formule I selon la définition dans la revendication 1, avec $R^1$ = $R^3$ = Y = H, $R^2$ = $CH_3$, X = O, Z = O en position 4 par rapport au groupe $XCH_2$, et

Ar = 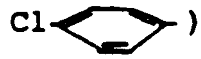.

**8.** 6-(biphénylyl-4-oxyméthyl)-1-hydroxy-4-méthyl-2-pyridone (= compose de formule I selon la définition dans la revendication 1, avec $R^1$ = $R^3$ = Y = H, $R^2$ = $CH_3$, X = O, Z représente une simple liaison et Ar = $C_6H_5$ en position 4 par rapport au groupe $XCH_2$).

**9.** 1-hydroxy-4-méthyl-6-[4-(4-trifluorométhylphénoxy)-phénoxyméthyl]-2-pyridone (= composé de formule I selon la définition dans la revendication 1, avec $R^1$ = $R^3$ = Y = H, $R^2$ = $CH_3$, X = O, Z = O en position 4 par rapport au groupe $XCH_2$, et

Ar = 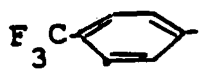.

**10.** Procédé pour la préparation de composés de formule I selon une ou plusieurs des revendications 1 à 9, caractérisé en ce que

a) on fait réagir une 6-halogénométhyl-2-pyrone de formule III

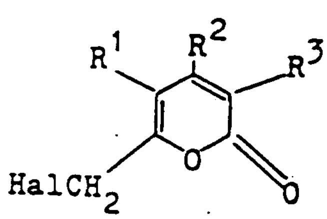

III

avec un phénol ou thiophénol de formule IV

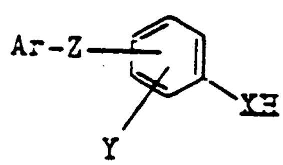

IV

et on convertit les aryloxyméthylpyrones ou arylthiométhylpyrones de formule V ainsi formées,

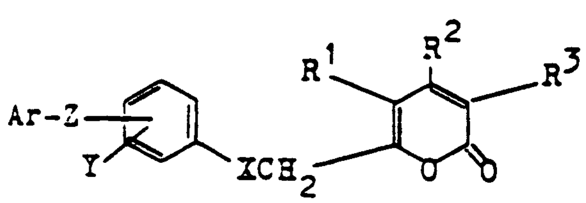

V

sous l'effet de l'hydroxylamine, en les hydroxypyridones de formule I, ou en ce que

b) on fait réagir une dihalogénopicoline de formule VI

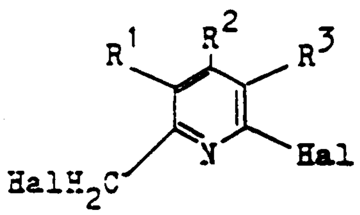

VI

avec un phénol de formule IV, on oxyde en N-oxyde le composé obtenu et on convertit le composé en un composé de formule I par saponification de l'halogène fixé au noyau,

dans les formules I et III à VI, les radicaux $R^1$, $R^2$, $R^3$, X, Y, Z et Ar ayant chacun la signification indiquée dans les revendications 1 à 9, et Hal représentant un atome d'halogène, en particulier de chlore ou de brome, ou en ce que, dans les composés qui ont été obtenus selon le mode opératoire a) ou b), ou dans les produits intermédiaires qui contiennent encore des groupes réactifs dans le cycle Ar ou dans le cycle pyridone, on introduit par échange de ceux-ci encore d'autres substituants, qui satisfont aux définitions des radicaux $R^1$, $R^2$, $R^3$ et des substituants de Ar.

**11.** Procédé selon la revendication 10, caractérisé en ce que, dans la variante a), on effectue la conversion des aryloxyméthylpyrones ou arylthiométhylpyrones de formule V en les hydroxypyridones de formule I par réaction avec au moins environ 1 mole d'un sel d'hydroxylammonium par mode de composé de formule V, en présence d'au moins environ 1 équivalent d'au moins une base organique ou minérale, par rapport au sel d'hydroxylammonium.

**12.** Procédé selon la revendication 11, caractérisé en ce que, en tant que bases, on utilise les carbonates et/ou hydrogénocarbonates des métaux alcalins, de préférence du sodium et/ou du potassium, en particulier $Na_2CO_3$.

**13.** Procédé selon la revendication 12, caractérisé en ce que, en tant que sel d'hydroxylammonium, on utilise le sulfate d'hydroxylammonium.

**14.** Composés de formule I selon une ou plusieurs des revendications 1 à 9, ou tels qu'obtenus selon une ou plusieurs des revendications 10 à 13, et/ou au moins un sel physiologiquement acceptable d'un tel composé avec une base organique ou minérale, pour utilisation en tant que médicament.

**15.** Médicament, contenant une quantité efficace d'au moins un composé de formule I selon une ou plusieurs des revendications 1 à 9 ou tel qu'obtenu selon une ou plusieurs des revendications 10 à 13, et/ou au moins un sel physiologiquement acceptable d'un tel composé avec une base organique ou minérale, en plus d'un véhicule physiologiquement acceptable et éventuellement d'autres additifs et/ou adjuvants.

**16.** Médicament selon la revendication 15, pour utilisation en tant qu'agent antimycosique, en particulier contre des champignons pathogènes de la peau et des muqueuses.

**17.** Procédé pour la fabrication d'un médicament selon la revendication 15 ou 16, caractérisé en ce que l'on met sous une forme pharmaceutique appropriée au moins un composé de formule I selon une ou plusieurs des revendications 1 à 9 ou tel qu'obtenu selon une ou plusieurs des revendications 10 à 13 et/ou au moins un sel physiologiquement acceptable d'un tel composé avec une base organique ou minérale, avec un véhicule physiologiquement acceptable et éventuellement d'autres additifs et/ou adjuvants.

**18.** Composés de formule V

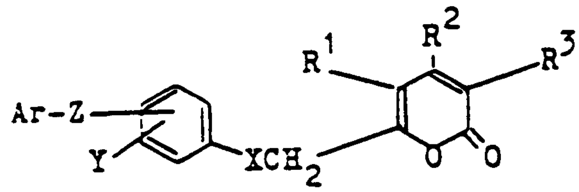

V

dans laquelle $R^1$, $R^2$, $R^3$, X, Y et Ar ont les significations données dans les revendications 1 à 9.

**19.** Procédé pour la préparation des composés de formule V selon la revendication 18, caractérisé en ce que l'on fait réagir une 6-halogénométhyl-2-pyrone de formule III

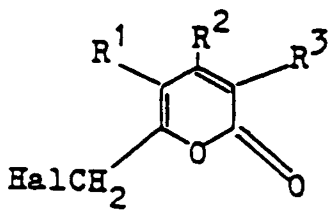

III

avec un phénol ou thiophénol de formule IV

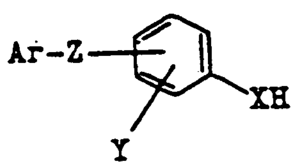

dans les formules, les radicaux $R^1$, $R^2$, $R^3$, X, Y, Z et Ar ayant les significations données dans les revendications 1 à 9, et
Hal représentant un atome d'halogène, en particulier de chlore ou de brome.

**Revendications pour les Etats contractants suivants : AT, ES, GR**

**1.** Procédé pour la préparation de composés de formule I

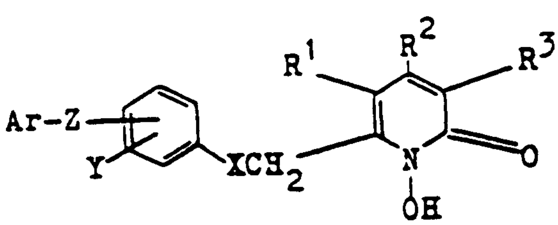

I

dans laquelle

| | |
|---|---|
| $R^1$, $R^2$ et $R^3$, | qui sont identiques ou différents, représentent un atome d'hydrogène ou un groupe alkyle inférieur ayant de 1 à 4 atomes de carbone, |
| X | représente S ou O, |
| Y | représente un atome d'hydrogène ou jusqu'à 2 atomes d'halogène, à savoir de chlore et/ou de brome, |
| Z | représente une simple liaison ou les radicaux bivalents O, S, $-CR_2-$ (R = H ou un groupe alkyle en $C_1$-$C_4$) ou d'autres radicaux bivalents ayant 2-10 atomes de |

carbone et éventuellement d'oxygène et/ou de soufre liés en formant une chaîne, lorsque les radicaux contiennent 2 atomes d'oxygène et/ou de soufre, ou plus, ces derniers devant être séparés l'un de l'autre (les uns des autres) par au moins 2 atomes de carbone, et 2 atomes de carbone contigus pouvant également être liés l'un à l'autre par une double liaison et les valences libres des atomes de carbone étant saturées par H et/ou des groupes alkyle en $C_1$-$C_4$,

Ar représente un système cyclique aromatique comportant jusqu'à deux cycles, choisi parmi les systèmes phényle, naphtyle, tétrahydronaphtyle, indényle, biphényle, diphénylalcane, éther diphénylique et diphénylthioéther, qui peut être substitué par jusqu'à trois substituants choisis parmi des atomes de fluor, chlore et brome et des groupes méthoxy, alkyle en $C_1$-$C_4$, trifluorométhyle et trifluorométhoxy,

caractérisé en ce que

a) on fait réagir une 6-halogénométhyl- 2-pyrone de formule III

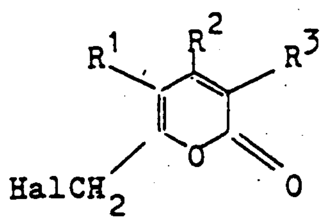

III

avec un phénol ou thiophénol de formule IV

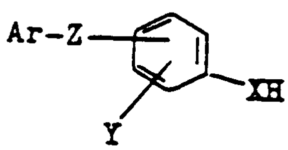

IV

et on convertit les aryloxyméthylpyrones ou arylthiométhylpyrones de formule V

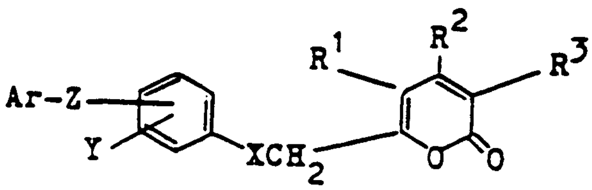

V

sous l'effet de l'hydroxylamine, en les hydroxypyridones de formule I, ou en ce que

b) on fait réagir une dihalogénopicoline de formule VI

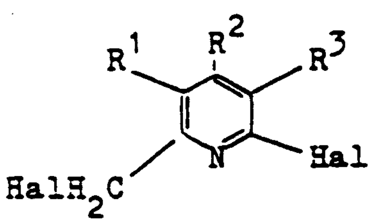

VI

avec un phénol de formule IV, on oxyde en N-oxyde le composé obtenu et on convertit le composé en un composé de formule I par saponification de l'halogène fixé au noyau,

dans les formules I et III à VI, les radicaux $R^1$, $R^2$, $R^3$, X, Y, Z et Ar ayant les mêmes significations que

dans la formule I, et Hal représentant un atome d'halogène, en particulier de chlore ou de brome, ou en ce que, dans les composés qui ont été obtenus selon le mode opératoire a) ou b), ou dans les produits intermédiaires qui contiennent encore des groupes réactifs dans le cycle Ar ou dans le cycle pyridone, on introduit par échange de ceux-ci encore d'autres substituants qui satisfont aux définitions des radicaux $R^1$, $R^2$, $R^3$ et des substituants de Ar.

**2.** Procédé selon la revendication 1, caractérisé en ce que l'on prépare des composés de formule I dans lesquels Ar représente le cycle phényle, éventuellement substitué.

**3.** Procédé selon la revendication 1, caractérisé en ce que l'on prépare des composés de formule I dans lesquels Ar représente un système bicyclique isolé qui dérive de préférence du biphényle, d'un diphénylalcane ou de l'éther diphénylique et éventuellement est substitué.

**4.** Procédé selon une ou plusieurs des revendications 1 à 3, caractérisé en ce que l'on prépare des composés de formule I dans lesquels Z est une simple liaison.

**5.** Procédé selon une ou plusieurs des revendications 1 à 3, caractérisé en ce que l'on prépare des composés de formule I dans lesquels Z représente ou contient un atome d'oxygène.

**6.** Procédé selon une ou plusieurs des revendications 1 à 3, caractérisé en ce que l'on prépare des composés de formule I dans lesquels $R^1$ et $R^3$ sont des atomes d'hydrogène, $R^2$ est le groupe méthyle et X est un atome d'oxygène.

**7.** Procédé selon la revendication 1, caractérisé en ce que l'on prépare la 6-[4-(4-chlorophénoxy)-phénoxyméthyl]-1-hydroxy-4-méthyl-2-pyridone (= composé de formule I selon la définition dans la revendication 1, avec $R^1$ = $R^3$ = Y = H, $R^2$ = $CH_3$, X = O, Z = O en position 4 par rapport au groupe $XCH_2$, et

Ar = 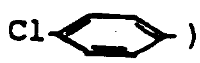

**8.** Procédé selon la revendication 1, caractérisé en ce que l'on prépare la 6-(biphénylyl-4-oxyméthyl)-1-hydroxy-4-méthyl-2-pyridone (= composé de formule I selon la définition dans la revendication 1, avec $R^1$ = $R^3$ = Y = H, $R^2$ = $CH_3$, X = O, Z représente une simple liaison et Ar = $C_6H_5$ en position 4 par rapport au groupe $XCH_2$).

**9.** Procédé selon la revendication 1, caractérisé en ce que l'on prépare la 1-hydroxy-4-méthyl-6-[4-(4-trifluorométhyl-phénoxy)-phénoxyméthyl]-2-pyridone (= composé de formule I selon la définition dans la revendication 1, avec $R^1$ = $R^3$ = Y = H, $R^2$ = $CH_3$, X = O, Z = O en position 4 par rapport au groupe $XCH_2$, et

Ar = 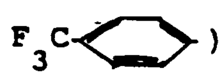

**10.** Procédé selon une ou plusieurs des revendications 1 à 9, caractérisé en ce que, dans la variante a), on effectue la conversion des aryloxyméthylpyrones ou arylthiométhylpyrones de formule V en les hydroxypyridones de formule I par réaction avec au moins environ 1 mole d'un sel d'hydroxylammonium par mole de composé de formule V, en présence d'au moins environ 1 équivalent d'au moins une base organique ou minérale, par rapport au sel d'hydroxylammonium.

**11.** Procédé selon la revendication 10, caractérisé en ce que, en tant que bases, on utilise les carbonates et/ou hydrogénocarbonates des métaux alcalins, de préférence du sodium et/ou du potassium, en particulier $Na_2CO_3$.

**12.** Procédé selon la revendication 11, caractérisé en ce que, en tant que sel d'hydroxylammonium, on utilise le sulfate d'hydroxylammonium.

**13.** Procédé pour la fabrication d'un médicament, caractérisé en ce que l'on met sous une forme pharmaceutique appropriée au moins un composé de formule I tel qu'obtenu selon une ou plusieurs des revendications 1 à 12, et/ou au moins un sel physiologiquement acceptable d'un tel composé avec une base organique ou minérale,
avec un véhicule physiologiquement acceptable et éventuellement d'autres additifs et/ou adjuvants.

**14.** Composés de formule V

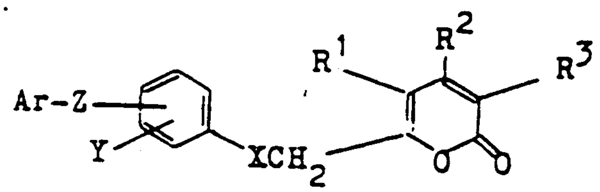

V

dans laquelle $R^1$, $R^2$, $R^3$, X, Y et Ar ont les significations données dans les revendications 1 à 9.

**15.** Procédé pour la préparation des composés de formule V selon la revendication 14, caractérisé en ce que l'on fait réagir une 6-halogénométhyl-2-pyrone de formule III

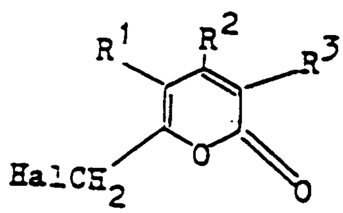

III

avec un phénol ou thiophénol de formule IV

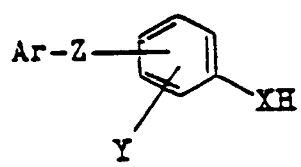

dans les formules, les radicaux $R^1$, $R^2$, $R^3$, X, Y, Z et Ar ayant les significations données dans les revendications 1 à 9, et
Hal représentant un atome d'halogène, en particulier de chlore ou de brome.